(19) Europäisches Patentamt
European Patent Office
Office européen des brevets

(11) **EP 2 763 720 B1**

(12) **FASCICULE DE BREVET EUROPEEN**

(45) Date de publication et mention
de la délivrance du brevet:
**04.11.2015 Bulletin 2015/45**

(21) Numéro de dépôt: **12775785.4**

(22) Date de dépôt: **28.09.2012**

(51) Int Cl.:
***A61M 1/34*** *(2006.01)*

(86) Numéro de dépôt international:
**PCT/FR2012/052198**

(87) Numéro de publication internationale:
**WO 2013/050689 (11.04.2013 Gazette 2013/15)**

(54) **MACHINE DE DIALYSE COMPRENANT DES MOYENS D'ULTRAFILTRATION ET DE RÉTROFILTRATION**

DIALYSEMASCHINE MIT ULTRAFILTRIERUNG UND RÜCKFILTRIERUNG

DIALYSIS MACHINE INCLUDING ULTRAFILTRATION AND BACKFILTRATION MEANS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.10.2011 FR 1158915**

(43) Date de publication de la demande:
**13.08.2014 Bulletin 2014/33**

(73) Titulaire: **Physidia**
**49124 Saint-Barthélémy-d'Anjou (FR)**

(72) Inventeur: **VINCENT, Eric**
**49000 Nantes (FR)**

(74) Mandataire: **Dutreix, Hugues Ours et al**
**Ipsilon Brema-Loyer**
**3, rue Edouard Nignon**
**44300 Nantes (FR)**

(56) Documents cités:
**EP-A2- 0 204 174      US-A1- 2001 035 378**
**US-A1- 2002 023 879      US-A1- 2009 008 306**

**Description**

La présente invention concerne de manière générale les machines de dialyse, notamment pour l'hémodialyse.

L'invention concerne plus particulièrement une machine de dialyse permettant de traiter un liquide corporel, tel que du sang ou du plasma. Ladite machine comporte un dialyseur comprenant une enceinte logeant une membrane de dialyse qui sépare l'enceinte en un compartiment de liquide corporel, et en un compartiment de dialysat. Ledit compartiment de liquide corporel, respectivement ledit compartiment de dialysat, présente une entrée de liquide corporel, respectivement une entrée de dialysat, et une sortie de liquide corporel, respectivement une sortie de dialysat. Ladite machine comporte aussi un système d'amenée du dialysat comprenant une ligne d'amenée de dialysat raccordée à l'entrée du compartiment de dialysat, et un système d'évacuation du dialysat comprenant une ligne d'évacuation de dialysat raccordée à la sortie du compartiment de dialysat. Ladite machine comprend aussi une unité de pilotage, telle qu'un automate programmable, qui comprend des moyens de commande d'ultrafiltration permettant de faire passer, à travers au moins une partie de la membrane, une quantité aqueuse du liquide corporel depuis le compartiment de liquide corporel jusque dans le compartiment de dialysat, et des moyens de commande de rétrofiltration permettant de faire passer le dialysat, à travers au moins une partie de la membrane, depuis le compartiment de dialysat jusque dans le compartiment de liquide corporel.

De manière générale, l'hémodialyse consiste à épurer le sang de substances présentes en excès, telles que l'urée, la vitamine B12, la beta-immunoglobuline, ou encore les sels minéraux. L'élimination de ces substances en excès peut s'effectuer par osmose en faisant circuler le sang d'un côté de la membrane et le dialysat de l'autre côté de la membrane. Ainsi, les substances présentes en excès dans le sang se diffusent à travers la membrane dans le dialysat.

Il est aussi possible d'extraire une fraction aqueuse du sang selon une opération appelée ultrafiltration. L'ultrafiltration résulte d'un phénomène de convection à travers la membrane. Sous l'effet d'une pression dans le compartiment sang supérieure à la pression dans le compartiment dialysat, une fraction aqueuse du sang est poussée à travers la membrane, ce qui diminue le volume de sang. Le sang est composé d'eau, de sels minéraux, de nombreuses substances et de cellules comme les globules rouges. Ladite fraction aqueuse du sang que ce mécanisme de convection permet de faire passer à travers la membrane, comprend notamment de l'eau, des sels minéraux et des molécules de poids moléculaires petit ou moyen, telles que la vitamine B12.

A l'inverse d'une opération d'ultrafiltration, il est aussi possible d'introduire une quantité appropriée de dialysat dans le sang selon une opération appelée rétrofiltration. Généralement, le dialysat sert alors à la fois de liquide d'échange avec le sang et de liquide de substitution directement introduit dans le circuit sanguin du patient.

On connaît de l'état de la technique, et notamment du document US5011607, une machine de dialyse telle que décrite ci-dessus. Cependant, dans le document US5011607, les moyens de commande de rétrofiltration sont complexes et peu précis puisqu'ils nécessitent la présence d'au moins une pompe en amont du compartiment dialysat et d'au moins une pompe en aval du compartiment dialysat. La pression dans le compartiment dialysat étant fonction de la différence entre les débits imposés par ces pompes amont et aval, il est nécessaire de piloter précisément les débits de ces deux pompes, ce qui complique le pilotage de la machine et augmente le risque d'erreurs. Or, le débit de dialysat rétrofiltré doit pouvoir être régulé avec une grande précision pour ne pas mettre en danger la vie du patient.

Le fait de devoir piloter une pompe amont et une pompe aval dans le circuit de dialysat pour rétrofiltrer une quantité donnée de dialysat nécessite de réguler le débit de chacune de ces pompes et pour ce faire de mesurer les débits correspondants amont et aval dans le circuit de dialysat. Il en résulte que l'erreur de mesure de débit associée à la pompe en amont est cumulée avec l'erreur de mesure de débit associée à la pompe en aval. En outre, on cumule aussi les erreurs de commande de débit associées à ces deux pompes.

Enfin, les pompes usuellement utilisées pour faire circuler le dialysat sont des pompes de type péristaltique qui présentent une faible précision de fonctionnement. En effet, une pompe péristaltique utilise des rouleaux qui écrasent un tuyau pour faire s'écouler le dialysat. Non seulement, la commande de débit avec de tels rouleaux n'est pas précise, mais en outre le tuyau se détériore rapidement de sorte que le débit de dialysat ne peut pas être déterminé précisément et de manière fiable. Ainsi, une telle pompe ne permet pas de commander un débit donné de dialysat avec une précision suffisante dans le cas d'une opération de rétrofiltration pour laquelle le débit de dialysat rétrofiltré doit pouvoir être déterminé avec précision et de manière fiable pour ne pas mettre en danger la vie du patient.

Il est aussi connu pour faire circuler le dialysat d'utiliser des pompes iso-volumes. Cependant, de telles pompes font circuler, à chaque activation de la pompe, un volume donné prédéfini de dialysat, ce qui ne permet pas d'obtenir un débit de dialysat suffisamment précis et régulier en entrée dans le compartiment dialysat au cours d'une étape d'ultrafiltration et/ou de rétrofiltration.

En outre, avec de telles machines connues de l'état de la technique, il est nécessaire de procéder à des opérations de nettoyage, stérilisation et rinçage de tous les composants du circuit dialysat qui ont été en contact avec le dialysat, tels que les tuyaux des pompes et les débitmètres. En effet, lorsque plusieurs patients sont traités, on change entre chaque séance de dialyse le filtre dialyseur, on jette les lignes d'accès sanguin qui relient le patient au dialyseur côtés artériel et veineux, et on nettoie, stérilise chimiquement ou thermiquement, et rince les tuyauteries de la machine, en particulier les lignes amont et aval de dialysat et les organes traversés par le dialysat tels que les pompes et les vannes.

Ces opérations sont longues, coûteuses et les risques de contamination restent importants.

Enfin, il est souhaitable de pouvoir améliorer le traitement proposé dans le document US5011607. En effet le pilotage des opérations de rétrofiltration et d'ultrafiltration proposées dans ce document est limité au fait de retirer un volume donné de fraction aqueuse du sang du patient par ultrafiltration et de remplacer ce volume par un même volume de dialysat.

Une autre machine de dialyse selon l'art antérieur est connue du document US2009/008306 A1.

La présente invention a pour but de proposer une nouvelle machine de dialyse permettant de commander des opérations d'ultrafiltration et de rétrofiltration de manière simple, précise et fiable. En particulier, un but de l'invention est de permettre de commander avec précision et de manière fiable le débit de dialysat qui entre dans le compartiment de dialysat.

La présente invention a aussi pour but de proposer une nouvelle machine de dialyse simplifiée, de coût réduit et grâce à laquelle le risque de contamination du liquide corporel à traiter est très faible.

A cet effet, l'invention a pour objet une machine de dialyse permettant de traiter un liquide corporel, tel que du sang ou du plasma, ladite machine comportant :

- un dialyseur comprenant une enceinte logeant une membrane de dialyse qui sépare l'enceinte en un compartiment de passage dudit liquide corporel, appelé compartiment de liquide corporel, et en un compartiment de passage de dialysat, appelé compartiment de dialysat,
- ledit compartiment de liquide corporel, respectivement ledit compartiment de dialysat, présentant une entrée de liquide corporel, respectivement une entrée de dialysat, et une sortie de liquide corporel, respectivement une sortie de dialysat,

- un système d'amenée du dialysat comprenant une ligne d'amenée de dialysat raccordée à l'entrée du compartiment de dialysat, et
- un système d'évacuation du dialysat comprenant une ligne d'évacuation de dialysat raccordée à la sortie du compartiment de dialysat,
- une unité de pilotage, telle qu'un automate programmable, qui comprend :

  - des moyens de commande d'ultrafiltration permettant de faire passer, à travers au moins une partie de la membrane, une quantité aqueuse du liquide corporel depuis le compartiment de liquide corporel jusque dans le compartiment de dialysat, et
  - des moyens de commande de rétrofiltration permettant de faire passer le dialysat, à travers au moins une partie de la membrane, depuis le compartiment de dialysat jusque dans le compartiment de liquide corporel,

caractérisée en ce que, la ligne d'amenée de dialysat comportant une restriction de passage, ladite machine comprend des moyens de mesure de la différence de pression aux bornes de ladite restriction de passage,
en ce que ledit système d'amenée du dialysat comprend aussi :

- au moins une poche souple, appelée poche ventricule, destinée à contenir du dialysat et raccordée à ladite ligne d'amenée en amont de ladite restriction de passage, ladite poche ventricule étant contenue dans une enceinte, et
- des moyens de mise sous pression de la poche ventricule par pressurisation de ladite enceinte,

et en ce que ladite machine comprend un système d'ouverture/fermeture de la ligne d'évacuation de dialysat autorisant, à l'état d'ouverture, la circulation dans ladite ligne d'évacuation du dialysat présent en sortie du compartiment de dialysat et empêchant, à l'état de fermeture, la circulation dans ladite ligne d'évacuation du dialysat présent en sortie dudit compartiment de dialysat, lesdits moyens de commande de rétrofiltration étant configurés pour :
- commander ledit système d'ouverture/fermeture pour fermer la ligne d'évacuation, et
- à l'état fermé de ladite ligne d'évacuation, commander la pression appliquée à ladite poche ventricule en fonction de la différence de pression mesurée aux bornes de la restriction, pour obtenir un débit de dialysat donné.

Le fait, pour commander une opération de rétrofiltration, de fermer la ligne d'évacuation à l'aide dudit système de fermeture/ouverture, permet de piloter simplement les moyens de mise sous pression de la poche ventricule pour obtenir le débit souhaité de dialysat pendant un temps donné afin de rétrofiltrer la quantité de dialysat souhaitée.

En effet, comme la ligne d'évacuation est fermée, le dialysat, amené dans le compartiment dialysat par application d'une pression sur la poche ventricule, ne peut sortir dudit compartiment dialysat qu'au travers de la membrane dans le compartiment de liquide corporel. Il en résulte que le débit de dialysat qui s'écoule dans la ligne d'amenée correspond au débit de dialysat rétrofiltré.

Ainsi, contrairement aux solutions connues de l'état de la technique, il n'est pas nécessaire, lors de l'opération de rétrofiltration, de piloter une pompe située en aval de la sortie du compartiment dialysat pour ajuster les débits de dialysat amont et aval. En effet avec la solution selon l'invention, le débit aval est nul puisque la ligne d'évacuation est fermée et le débit de dialysat rétrofiltré correspond à celui entrant dans le compartiment dialysat.

Le pilotage de l'opération de rétrofiltration est ainsi simplifié et peut s'effectuer avec une grande précision puisque non seulement le débitmètre, formé par la restriction de passage et les moyens de mesure de différence de pression, permet d'obtenir un débit donné avec une grande précision, mais, en plus, le débit rétrofiltré ne dépend pas d'un éventuel débit en aval dans la ligne d'évacuation, ce qui permet de s'exempter des erreurs de précision d'une éventuelle pompe aval telle qu'utilisée dans les machines connues de l'état de la technique.

Il est à noter que, comme expliqué ci-dessus, l'utilisation d'une pompe péristaltique à la place des moyens de mise sous pression et de la poche souple ne permettrait pas d'imposer un débit de dialysat donné de manière stable et suffisamment précise. En effet, avec la machine selon l'invention, la réalisation de la pompe de dialysat sous forme d'une poche ventricule souple, logée dans une enceinte et associée à des moyens de pressurisation de l'enceinte, permet d'appliquer une pression à la poche de manière fiable et précise et ainsi, en coopération avec le débitmètre formé de la restriction de passage et des capteurs de pression correspondants, d'obtenir un débit de fluide plus précis que celui que l'on peut obtenir avec une pompe péristaltique qui utilise des rouleaux pour écraser une tubulure. De même, comme expliqué ci-dessus, l'utilisation d'une pompe iso-volume ne serait pas suffisamment précise pour obtenir le débit de dialysat souhaité.

La commande de la pression appliquée à la poche ventricule en fonction de la différence de pression mesurée aux bornes de la restriction permet de réguler ladite pression appliquée à la poche pour maintenir un débit donné pendant un temps donné afin d'obtenir une quantité de dialysat rétrofiltrée de manière précise et fiable.

En particulier, la commande de la pression appliquée à la poche et sa régulation à l'aide du débitmètre, formé par la restriction et les moyens de mesure de différence de pression associés, permet de maintenir la pression moyenne dans le compartiment de dialysat à une valeur donnée. Cette valeur est déterminée par l'unité de pilotage pour, compte tenu de la pression moyenne dans le compartiment sang et des caractéristiques de la membrane, rétrofiltrer un débit donné de dialysat pendant une durée donnée. En effet, la mesure de la différence de pression aux bornes de la restriction permet à l'unité de pilotage, et en particulier aux moyens de commande de rétrofiltration, de réguler la commande de la pression appliquée à la poche ventricule pour maintenir pendant ladite durée de rétrofiltration la pression dans le compartiment de dialysat, et donc le débit de dialysat rétrofiltré, à une valeur donnée.

Quand le système d'ouverture/fermeture de la ligne d'évacuation est en position d'ouverture, le dialysat peut s'écouler depuis la poche ventricule à travers la ligne d'amenée, le compartiment de dialysat et la ligne d'évacuation. Lorsque les moyens de commande d'ultrafiltration commandent l'ouverture de la ligne d'évacuation, la pression moyenne dans le compartiment de dialysat, générée par le dialysat qui s'écoule de la poche ventricule, est maintenue inférieure à la pression moyenne dans le compartiment de liquide corporel de sorte qu'une fraction aqueuse du liquide corporel passe du compartiment de liquide corporel vers le compartiment dialysat, entrainant de l'eau et certaines toxines urémiques.

En outre, la conception du système d'amenée de dialysat sous forme d'au moins une poche ventricule et de moyens de pressurisation, ainsi que la présence d'une restriction de passage dans la ligne d'amenée pour former, en combinaison avec des moyens de mesure de différence de pression, un débitmètre, permet de concevoir le circuit de dialysat sous forme d'un circuit jetable dont les composants en contact avec le dialysat sont simples et peu coûteux, ce qui permet de les remplacer pour chaque séance et ainsi d'assurer la continuité de la stérilité de la machine d'une séance à une autre. Comme détaillé ci-après, les autres composants, tels que les moyens de mesure de la différence de pression et le système d'ouverture/fermeture de la ligne d'évacuation, sont agencés de manière à ne pas être en contact avec le dialysat.

En particulier, les lignes de dialysat et poches peuvent être jetées après la séance, ce qui supprime le besoin de stériliser et rincer la machine. Il n'est pas nécessaire de stériliser les moyens de mise sous pression puisque ceux-ci appliquent une pression gazeuse sur la paroi de la ou de chaque poche et ne sont pas en contact direct avec le liquide. La poche ventricule peut être aisément jetée et remplacée en ouvrant l'enceinte dans laquelle elle est logée. Le dialyseur, les lignes de dialysat et de sang, les poches, et les tubes à perte de charge sont économiques et peuvent ainsi être à usage unique.

Selon une caractéristique avantageuse de l'invention, ledit système d'évacuation du dialysat comprend au moins une poche de décharge, de préférence souple, présentant une entrée et une sortie raccordées à la ligne d'évacuation de dialysat,

et ledit système d'ouverture/fermeture comprend un organe amont d'ouverture/fermeture situé entre la sortie du compartiment dialysat et ladite poche de décharge,

et ladite machine comprend des moyens de détermination de la quantité de dialysat et de liquide récupérée dans ladite poche de décharge.

Ladite poche de décharge permet, en configuration ouverte de la ligne d'évacuation, de récupérer le dialysat écoulé de la poche ventricule et la fraction aqueuse extraite du liquide corporel par ultrafiltration, de manière à déterminer la quantité aqueuse extraite du liquide corporel. Il suffit en effet de soustraire de la quantité totale récupérée dans la poche de décharge, la quantité de dialysat écoulée de la poche ventricule pour déterminer ladite quantité aqueuse extraite du liquide corporel. La connaissance de cette quantité aqueuse permet de calculer la quantité de liquide à rétrofiltrer et éventuellement d'ajuster la quantité aqueuse à extraire et/ou à rétrofiltrer au cours d'un prochain cycle.

Préférentiellement, ledit système d'évacuation du dialysat comprend au moins une autre poche de décharge montée en dérivation de ladite poche de décharge,
et ledit système d'ouverture/fermeture aval comprend un autre organe amont d'ouverture/fermeture situé entre ladite autre poche de décharge et le noeud d'entrée de raccordement des poches de décharge,
et ladite machine comprend des moyens de détermination de la quantité de dialysat et de liquide récupérée dans ladite autre poche de décharge.

La présence de deux poches de décharge montées en parallèle permet de limiter les temps morts dans le fonctionnement de la machine, puisqu'il est ainsi possible de vider l'une des poches de décharge précédemment remplie pendant que l'autre se remplit.

Avantageusement, lesdits moyens de détermination de la quantité de dialysat et de liquide récupérée dans la ou les poches de décharge comprennent une restriction de passage ménagée dans la ligne d'évacuation de dialysat et située en aval de la ou desdites poches de décharge, et des moyens de mesure de la différence de pression aux bornes de ladite restriction de passage.

De tels moyens de mesure de la différence de pression aux bornes de ladite restriction de passage de la ligne d'évacuation permettent de déterminer le débit évacué de ladite ou de l'une desdites poches de décharge et ainsi de déterminer, en mesurant le temps d'écoulement correspondant, la quantité de dialysat et de liquide récupérée dans ladite poche de décharge.

La ou chaque poche ventricule et la ou chaque poche de décharge sont distinctes l'une de l'autre de sorte que, lorsque l'organe amont d'ouverture/fermeture, associé à la ou l'une des poches de décharge, est en position fermée, la ou l'une des poches ventricules peut être pressurisée sans que cela ait d'influence sur ladite poche de décharge. Autrement dit, lesdites poches n'ont pas de paroi commune ou en contact direct. Une telle conception des poches permet de vider la ou chaque poche de décharge en temps masqué pendant une étape d'ultrafiltration ou de rétrofiltration.

Selon une caractéristique avantageuse de l'invention, lesdits moyens de mesure de la différence de pression aux bornes de la restriction de la ligne d'amenée, respectivement aux bornes de la restriction de la ligne d'évacuation lorsque ladite restriction est présente, comprennent :

-   deux orifices, dits orifices de prise de pression, ménagés l'un en amont et l'autre en aval de ladite restriction, et,
-   pour chaque orifice de prise de pression, un capteur de pression agencé de manière à mesurer la pression dans la ligne correspondante au niveau dudit orifice tout en étant écarté dudit orifice pour ne pas être en contact avec le dialysat circulant dans la ligne.

Il est ainsi possible de jeter les lignes de dialysat tout en conservant les capteurs puisque ceux-ci sont à l'abri du dialysat. Il en résulte que le coût d'usage de la machine est réduit tout en conservant un risque de contamination très faible.

Préférentiellement, un filtre perméable à l'air et imperméable aux agents infectieux et aux liquides, est interposé entre chaque capteur de pression et l'orifice de prise de pression correspondant.

Ledit filtre permet, en cas de remonté de gouttes de dialysat par l'orifice de prise de pression correspondant, d'intercepter ces gouttes et ainsi de préserver le capteur d'un éventuel risque de contamination.

Avantageusement, ladite machine présentant un bâti, lesdits capteurs de pression sont fixés au bâti de la machine, et les lignes d'amenée de dialysat et d'évacuation de dialysat sont aptes à être retirées du reste de la machine, tandis que les capteurs de pression correspondants restent fixés au bâti de la machine.

Comme rappelé ci-dessus, entre chaque séance de dialyse, on jette et on remplace le dialyseur et les lignes d'accès sanguin du côté artériel et veineux. On jette et on remplace aussi les poches ventricules et de décharge, ainsi que les lignes d'amenée et d'évacuation. Mais les capteurs de pression restent en place dans la machine. En effet, au cours de la séance de traitement précédente, lesdits capteurs de pression sont restés à l'abri du dialysat grâce à leur écartement de l'orifice de prise de pression, et, de préférence, au filtre intégré à un conduit interposé entre le capteur et ledit orifice de prise de pression. Il n'est donc plus nécessaire avec la machine selon l'invention de stériliser chimiquement ou thermiquement, et de rincer les tuyauteries de la machine. En effet, les composants de la machine qui ont été en contact avec le dialysat sont peu coûteux et sont séparables du bâti de la machine, ce qui permet de les jeter et de les remplacer aisément.

En outre, le fait d'intégrer les capteurs de pression dans le bâti de la machine permet de concevoir une machine compacte.

Selon une caractéristique avantageuse de l'invention, pour commander la fermeture de la ligne d'évacuation de dialysat en vue d'une étape de rétrofiltration, lesdits moyens de commande de rétrofiltration sont configurés pour commander la fermeture de chacun desdits organes amont d'ouverture/fermeture associés aux poches de décharge, et, pour commander l'ouverture de la ligne d'évacuation de dialysat en vue d'une étape d'ultrafiltration, lesdits moyens de commande d'ultrafiltration sont configurés pour commander l'ouverture de l'un desdits organes amont d'ouverture/fermeture et la fermeture de l'autre organe amont d'ouverture/fermeture,

lesdits moyens de commande d'ultrafiltration étant de préférence configurés pour, à l'étape d'ultrafiltration suivante, commander l'ouverture de l'autre desdits organes amont d'ouverture/fermeture et la fermeture dudit organe amont d'ouverture/fermeture.

Ainsi, les poches de décharge sont remplies en alternance, ce qui permet de vider l'une pendant que l'autre se remplit.

Avantageusement, la ou chaque poche de décharge est munie d'un organe, dit aval, d'ouverture/fermeture situé en aval de ladite poche,

et, après une étape d'ultrafiltration, ladite unité de pilotage est configurée pour commander, de préférence en parallèle d'une étape de rétrofiltration, l'ouverture de l'organe aval d'ouverture/fermeture de la poche de décharge précédemment remplie afin d'évacuer son contenu,

et, de préférence, pour déterminer la quantité récupérée dans ladite poche de décharge, l'unité de pilotage est configurée pour mesurer le débit qui s'écoule de ladite poche de décharge, à l'aide desdits moyens de mesure de la différence de pression aux bornes de la restriction située en aval de ladite poche de décharge.

Ladite restriction de la ligne d'évacuation est aussi située en aval de l'organe aval d'ouverture/fermeture associé à la ou chaque poche de décharge.

La mesure de la quantité de dialysat et de liquide récupérée dans une poche de décharge peut ainsi s'effectuer de manière fiable et en temps masqué en parallèle d'une étape d'ultrafiltration et/ou de rétrofiltration.

Selon une caractéristique avantageuse de l'invention, ladite unité de pilotage comprend aussi des moyens de détermination d'une quantité aqueuse en excès à retirer du liquide corporel, appelée quantité aqueuse en excès,

et les moyens de commande d'ultrafiltration sont configurés pour extraire à travers la membrane de dialyse une quantité aqueuse du liquide corporel, appelée quantité aqueuse brute à retirer, supérieure à ladite quantité aqueuse en excès définie,

les moyens de commande de rétrofiltration étant configurés pour rétrofiltrer une quantité de dialysat sensiblement égale à la différence entre ladite quantité aqueuse brute à retirer et ladite quantité aqueuse en excès.

Le fait de retirer du liquide corporel une quantité aqueuse supérieure à celle en excès et de compenser le surplus par injection de dialysat dans ledit liquide corporel, permet d'épurer ledit liquide corporel de manière rapide et efficace. Avantageusement, ladite unité de pilotage est configurée pour commander au cours d'une séance de traitement des étapes de rétrofiltration en alternance avec des étapes d'ultrafiltration, le nombre d'alternances étant de préférence au moins égal à 1000.

Une telle alternance d'étapes de rétrofiltration et d'ultrafiltration permet de traiter le liquide corporel rapidement et de manière homogène de sorte que ledit liquide corporel ainsi traité peut être réinjecté dans le corps du patient sans risque de déséquilibre de ses paramètres physiologiques. Une telle alternance permet aussi d'optimiser l'efficacité de la membrane en réduisant le risque de colmatage de la membrane et de coagulation lorsque ledit liquide corporel est du sang.

Selon une caractéristique avantageuse de l'invention, pour un nombre, noté N, de cycles, chaque cycle i, i allant de 1 à N, étant composé d'une étape d'ultrafiltration de durée $Te\_uf\_i$ et d'une étape de rétrofiltration de durée $Te\_rf\_i$,

ladite unité de pilotage est configurée pour :

- déterminer la quantité aqueuse en excès dans le liquide corporel, notée $Q\_aq\_exces$ qui doit être retirée;
- pour chaque cycle i, définir les durées $Te\_uf\_i$ et $Te\_rf\_i$, et piloter les moyens de mise sous pression de telle sorte que :

$$Q\_aq\_exces = \text{Somme sur } i, \text{ pour } i \text{ allant de 1 à N, de}$$

$$K*[(Pa\_i + Pv\_i)/2 - P14\_i]*Te\_uf\_i - (Dd\_rf\_i*Te\_rf\_i),$$

K étant un coefficient d'ultrafiltration prédéfini propre à la membrane qui traduit sa capacité d'ultrafiltration,
$Pa\_i$ étant la pression en amont du compartiment de liquide corporel au cours de l'étape d'ultrafiltration du cycle i,
$Pv\_i$ la pression en aval du compartiment de liquide corporel au cours de l'étape d'ultrafiltration du cycle i, et
$P14\_i$ la pression moyenne dans le compartiment de dialysat au cours de l'étape d'ultrafiltration du cycle i,

Dd_rf_i le débit de dialysat dans le compartiment de dialysat au cours de l'étape de rétrofiltration du cycle i.

Il est ainsi possible de maitriser la quantité aqueuse extraite du liquide corporel et la quantité de dialysat réinjectée en ajustant les durées Te_uf_i et Te_rf_i et la pression appliquée à la poche ventricule correspondante.

Selon une caractéristique avantageuse de l'invention, ledit système d'ouverture/fermeture de la ligne d'évacuation de dialysat comprend au moins une pince apte, d'une part, à pincer de l'extérieur la paroi d'une portion souple de ladite ligne d'évacuation de manière à fermer cette portion, et, d'autre part, à laisser ouverte ladite portion souple de ladite ligne d'évacuation.

L'ouverture/fermeture de ladite ligne d'évacuation à l'aide d'une pince permettant, d'une part, de pincer une portion souple de la ligne pour empêcher la circulation à travers cette portion et, d'autre part, de laisser libre cette portion pour autoriser ladite circulation, permet de commander rapidement et de manière fiable la fermeture de la portion correspondante de la ligne d'évacuation. En outre, l'utilisation d'une telle pince externe à la ligne d'évacuation permet de commander la fermeture/ouverture de la portion correspondante de la ligne d'évacuation sans risque de contamination de la pince puisque celle-ci n'est pas en contact avec le liquide présent dans ladite ligne d'évacuation. Ainsi, une telle pince peut être réutilisée sans avoir à être nettoyée contrairement à une solution qui utiliserait une vanne tout ou rien, ou proportionnelle, en contact avec le dialysat.

Selon une caractéristique avantageuse de l'invention, chacun des organes amont, et, le cas échéant, aval, de fermeture/ouverture de la ligne d'évacuation de dialysat comprend au moins une pince apte, d'une part, à pincer de l'extérieur la paroi d'une portion souple de ladite ligne d'évacuation de manière à fermer cette portion, et, d'autre part, à laisser ouverte ladite portion souple de ladite ligne d'évacuation.

Ainsi, le système de fermeture/ouverture de la ligne d'évacuation peut être réalisé de manière simple et peu coûteuse. En effet comme expliqué ci-dessus, chaque pince est isolée du liquide présent dans la ligne d'évacuation et n'a donc pas besoin d'être nettoyée ou jetée.

Selon une caractéristique avantageuse de l'invention, le système d'amenée du dialysat comprend une poche ventricule supplémentaire, les deux poches ventricules étant aptes à être mises sous pression séparément par lesdits moyens de mise sous pression. Chaque poche ventricule est logée dans une enceinte sensiblement étanche apte à être mise sous pression par lesdits moyens pneumatiques pour mettre sous pression ladite poche ventricule.

Ladite machine de dialyse comporte avantageusement des moyens d'ouverture et de fermeture alternative des conduits de raccordement des poches ventricules à la ligne d'amenée. Lesdits moyens d'ouverture et de fermeture alternative sont de préférence formés par un système de pinçage alternatif desdits conduits.

Une telle utilisation de deux poches ventricules montées en parallèle permet d'éviter une rupture de débit du dialysat et d'éviter des temps morts de fonctionnement de la machine. En effet, les poches ventricules sont utilisées en alternance de sorte que l'une des poches ventricules peut être remplie pendant que l'autre poche ventricule délivre le dialysat.

En outre, la conception du système de circulation amont et aval de dialysat sous forme de poches ventricules et d'enceintes sensiblement étanches, permet entre deux séances de remplacer aisément et rapidement les poches ventricules du circuit de dialysat par d'autres poches stériles en ouvrant simplement l'enceinte, afin de limiter le risque de contamination.

Selon une caractéristique avantageuse de l'invention, ladite ou chaque poche ventricule comprend aussi une entrée de raccordement à une source d'alimentation en dialysat, de préférence une poche nourricière de plus grand volume que la ou chaque poche ventricule.

Pour un traitement, appelé dialyse fréquente ou quotidienne, formé de séances plus courtes mais sur un plus grand nombre de jours par comparaison aux dialyses conventionnelles qui s'effectuent à l'hôpital, les volumes de dialysat nécessaires pour chaque séance sont plus faible de sorte que l'utilisation de poches nourricières contenant du dialysat prêt à l'emploi est particulièrement bien adaptée. Une telle dialyse fréquente ou quotidienne présente l'intérêt thérapeutique de minimiser les amplitudes des paramètres physiologiques, tels que le taux de concentration en toxines ou le taux de surpoids.

En effet, le fait de traiter le patient à intervalles relativement longs constitue un inconvénient important. Il stocke alors de manière importante entre les séances des toxines et de l'eau en excès, et son traitement nécessite une intervention longue et fatigante pour lui et un appareillage complexe en raison notamment des grandes quantités de dialysat ultrapur à préparer.

La dialyse quotidienne peut ainsi s'effectuer avec moins de dialysat par séance ce qui permet d'utiliser des poches et donc de s'exempter de machine de génération de dialysat en continu. La dialyse quotidienne peut ainsi être réalisée à domicile ce qui permet d'économiser le transport de la personne dialysée. Le dialysat étant prêt à l'emploi dans des poches, la charge de travail et de la surveillance liée à la préparation de dialysat est réduite.

L'invention sera bien comprise à la lecture de la description suivante d'exemples de réalisation, en référence aux dessins annexés dans lesquels :

- la figure 1 est un schéma simplifié de la machine de dialyse selon l'invention ;
- la figure 2 est une vue schématique d'un capteur de pression fixé au bâti de la machine et d'un conduit de prise de pression muni d'un filtre ;
- les figures 3 et 4 sont des vues schématiques illustrant respectivement une étape d'ultrafiltration et une étape de rétrofiltration ;
- la figure 5 est un schéma de la machine selon l'invention, complété par rapport à celui de la figure 1, qui montre notamment les deux poches ventricules et les deux poches de décharge,
- la figure 6 est un graphique illustrant, en fonction du temps, le débit transmembranaire qui résulte d'une pluralité de cycles d'ultrafiltration et de rétrofiltration ;
- la figure 7 est un graphique illustrant la configuration ouverte (1) ou fermée (0) de la ligne d'évacuation pour la pluralité de cycles d'ultrafiltration et de rétrofiltration de la figure 6.

En référence aux figures et comme rappelé ci-dessus, l'invention concerne une machine de dialyse permettant de traiter un liquide corporel, tel que du sang ou du plasma. Par liquide corporel, on entend un liquide du type de ceux présents dans le corps humain ou animal, tel que du sang ou du plasma.

Il doit être noté que la machine de dialyse selon l'invention est détaillée ci-dessous dans le cadre où le liquide corporel à épurer est du sang. Le sang est composé d'eau, de sels minéraux, de nombreuses substances et de cellules comme les globules rouges. Bien entendu, la description de la machine ci-dessous s'applique aussi à un liquide corporel autre que le sang.

Ladite machine comporte un dialyseur 1 comprenant une enceinte logeant une membrane 3 de dialyse qui sépare l'enceinte en un compartiment sang 12 et en un compartiment dialysat 14.

Le dialysat est un liquide bien connu de l'homme du métier dont la composition est proche du sérum physiologique. Le dialysat comprend de l'eau d'une qualité suffisante sur le plan minéral et sur le plan bactériologique. Cette eau purifiée est additionnée à un concentré, sous forme de poudre de sels minéraux ou sous forme de concentré liquide, pour fabriquer le dialysat requis. La circulation de dialysat passe par le compartiment du dialysat 14, généralement à contre-sens de la circulation sanguine dans l'autre compartiment 12. Le dialysat circule généralement en circuit ouvert. On peut aussi prévoir une boucle de régénération partielle de dialysat propre à partir du dialysat chargé des toxines.

La membrane 3 de dialyse semi-perméable est conçue de manière à laisser passer une fraction du volume sanguin lorsque la différence entre la pression locale dans le compartiment sang 12 et la pression locale dans le compartiment dialysat 14 est supérieure à une valeur donnée. Cette différence de pression est appelée pression transmembranaire. En particulier, la membrane est conçue de telle sorte que seules les molécules les plus petites peuvent passer à travers les trous ou pores de la membrane, à savoir l'eau, les sels minéraux et les molécules de petit à moyen poids moléculaire.

Plus précisément, ladite membrane 3 est conçue de telle sorte que, lorsque la différence entre la pression qui s'exerce sur au moins une portion de la membrane du côté du compartiment sang 12 et la pression qui s'exerce sur ladite portion de la membrane du côté du compartiment de dialysat 14, est supérieure à une valeur seuil donnée, ladite portion de la membrane laisse passer une fraction aqueuse du sang dans le compartiment de dialysat 14, selon un phénomène convectif appelé ultrafiltration. Inversement, ladite membrane 3 est conçue de telle sorte que, lorsque la différence entre la pression, qui s'exerce sur ladite portion de la membrane 3 du côté du compartiment de dialysat 14, et la pression, qui s'exerce sur ladite portion de la membrane du côté du compartiment sang 12, est supérieure à une valeur seuil donnée, ladite portion de la membrane laisse passer le dialysat dans le compartiment sang 12, selon un phénomène appelé rétrofiltration.

La différence entre la pression moyenne dans le compartiment sang et la pression moyenne dans le compartiment dialysat est appelée pression transmembranaire moyenne. Ladite moyenne s'effectue sur la surface de la membrane.

La membrane de dialyse peut être réalisée sous forme de fibres capillaires, le sang circulant à l'intérieur des fibres, le dialysat à l'extérieur.

Ledit compartiment sang 12, présente une entrée 201 de sang destinée à être raccordée par une ligne artérielle L1, à une fistule du patient, pour extraire le sang du corps du patient à traiter et une sortie 202 de sang, destinée à être raccordée par une ligne veineuse L2, à une veine du patient pour réintroduire le sang dans le corps du patient après traitement. On entend par "ligne" une tubulure comprenant éventuellement plusieurs branches ou portions et à travers laquelle un liquide peut circuler.

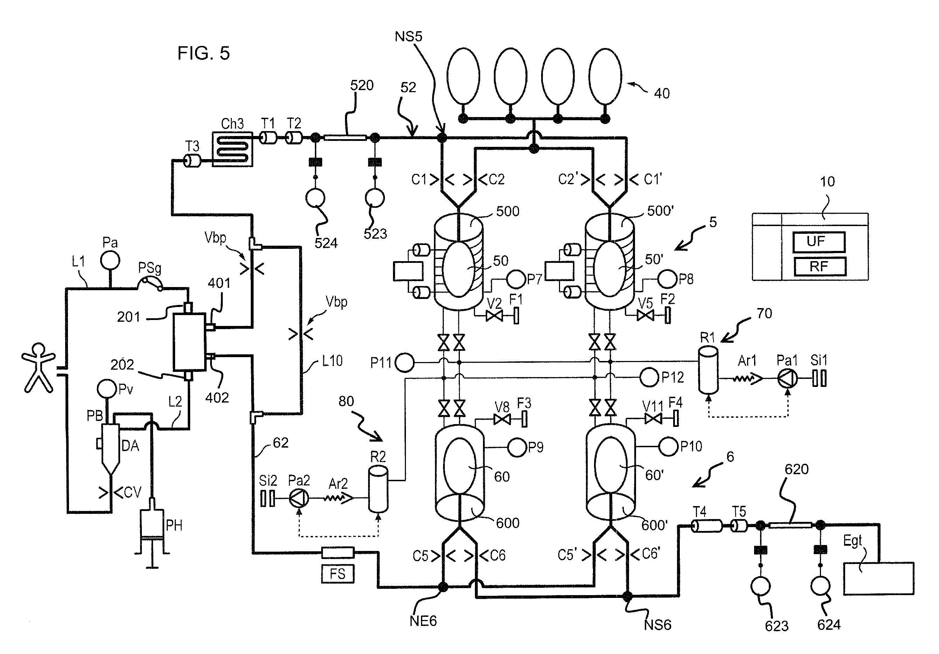

Dans l'exemple illustré à la figure 5, la ligne artérielle L1 est aussi munie d'une pompe à sang Psg, et d'un capteur de pression Pa, encore appelé capteur de pression artérielle. La ligne veineuse L2 comprend un capteur de pression Pv, encore appelé capteur de pression veineuse, un piège à bulle PB, et un détecteur d'air DA. Avantageusement, le capteur de pression Pv est connecté sur le piège à bulle PB. Préférentiellement, ladite ligne veineuse L2 comprend aussi un système d'injection d'anticoagulant, tel qu'une pompe à héparine PH.

La pression moyenne dans le compartiment sang 12 est due à la pression de retour veineux additionnée à la pression liée à la perte de charge dans le compartiment sang 12 qui dépend de la viscosité du sang et de son débit. La pression de retour veineux dépend de l'aiguille et de l'accès vasculaire utilisés, ainsi que de la pression du patient.

La ligne artérielle L1, la ligne veineuse L2 et le compartiment sang 12 forment le circuit sanguin de la machine.

Ledit compartiment de dialysat 14 présente une entrée de dialysat 401 et une sortie de dialysat 402. La machine comprend aussi un système d'amenée du dialysat 5 qui comporte une ligne d'amenée 52 de dialysat raccordée à l'entrée 401 du compartiment de dialysat 14, et un système d'évacuation du dialysat 6 qui comporte une ligne d'évacuation 62 raccordée à la sortie 402 du compartiment de dialysat 14. Ledit système d'amenée du dialysat 5, le compartiment de dialysat 14, et ledit système d'évacuation du dialysat 6 forment le circuit de dialysat.

Ladite machine comprend aussi une unité de pilotage 10, telle qu'un automate programmable.

Ladite unité de pilotage se présente sous la forme d'un système électronique et informatique qui comprend par exemple un microprocesseur et une mémoire de travail. Ladite unité de pilotage présente aussi une interface d'entrée de données, telles que le poids "sec" du patient et le poids dudit patient avant le traitement comme détaillé ci-après.

Ainsi, lorsqu'il est précisé que l'unité de pilotage comprend des moyens pour réaliser une action donnée, cela signifie que le système électronique et informatique correspondant comprend des instructions permettant d'exécuter ladite action.

Ladite unité de pilotage comprend des moyens de commande d'ultrafiltration UF et des moyens de commande de rétrofiltration RF. Comme rappelé ci-dessus, l'ultrafiltration correspond au passage, à travers au moins une partie de la membrane 3, d'une quantité aqueuse du liquide corporel depuis le compartiment sang 12 jusque dans le compartiment de dialysat 14. Inversement, la rétrofiltration correspond au passage du dialysat, à travers au moins une partie de la membrane 3, depuis le compartiment de dialysat 14 jusque dans le compartiment sang 12. Le fonctionnement desdits moyens de commande d'ultrafiltration UF et de rétrofiltration RF est détaillé ci-après

Ledit système d'amenée du dialysat 5 comprend aussi au moins une poche 50 souple, appelée poche ventricule, destinée à contenir du dialysat et raccordée à ladite ligne d'amenée 52.

Préférentiellement, comme illustré à la figure 5, ledit système d'amenée du dialysat 5 comprend une poche ventricule supplémentaire 50' qui est montée en dérivation de la portion de la ligne d'amenée 52 sur laquelle est raccordée ladite poche ventricule 50.

Chaque poche ventricule 50, 50' loge dans une enceinte 500, 500' sensiblement étanche qui peut être mise sous pression, et éventuellement en dépression. Chaque poche ventricule 50, 50' peut ainsi être mise sous pression par des moyens de mise sous pression 70 et, éventuellement, en dépression par un générateur de vide 80.

Lesdits moyens de mise sous pression 70 comprennent un dispositif d'injection de gaz sous pression, tel qu'un compresseur d'air ou une bouteille de gaz comprimé, apte à injecter du gaz sous pression dans l'enceinte 500, 500' étanche dans laquelle loge la poche ventricule 50, 50'. Ladite enceinte présente une sortie 501 dont l'ouverture est contrôlable par une électrovanne pour réduire la pression dans l'enceinte. Chaque enceinte 500, 500' est maintenue à une température appropriée pour que le dialysat stérile soit sensiblement à 37°C.

L'application d'une pression sur la poche ventricule 50 ou 50' et la régulation de cette pression appliquée, à l'aide de la différence de pression mesurée au bornes du tube à perte de charge 520 comme détaillé ci-après, permet de maintenir pendant un temps donné un débit fixe de dialysat dans le compartiment dialysat de manière fiable et précise, et ainsi de maîtriser les opérations de rétrofiltration comme expliqué ci-après. L'application et la régulation de la pression appliquée sur la poche ventricule 50 ou 50' permet aussi, en ajustant la pression appliquée à la poche de décharge 60 ou 60' comme détaillé ci-après, de maintenir pendant un temps donné une pression moyenne donnée dans le compartiment dialysat, et ainsi de maitriser les opérations d'ultrafiltration.

Les deux poches ventricules 50, 50' sont aptes à être mises sous pression/dépression indépendamment l'une de l'autre.

Ladite ou chaque poche ventricule 50, 50' comprend aussi une entrée de raccordement à une source d'alimentation en dialysat 40. Cette source d'alimentation en dialysat est de préférence une poche nourricière de plus grand volume, par exemple 5L, que celui, par exemple 150 ml, de ladite poche ventricule 50, 50'.

En variante, la source d'alimentation externe peut être un dispositif de production de dialysat en continu.

Comme illustré à la figure 5, chaque poche ventricule 50, 50' est aussi munie d'un organe aval de fermeture/ouverture C1, C1' de la ligne d'amenée qui, à l'état ouvert, autorise l'écoulement de dialysat depuis la poche 50, 50' correspondante vers l'entrée 401 du compartiment dialysat, sous l'effet d'une pression appliquée sur ladite poche ventricule par les moyens 70 correspondants, et à l'état fermé, empêche ledit écoulement de dialysat. Chaque organe aval de fermeture/ouverture C1, C1' de la ligne d'amenée est situé entre la poche correspondante 50, 50' et le noeud de sortie NS5 de raccordement des deux poches 50, 50'. Chaque poche ventricule 50, 50' est aussi munie d'un organe amont de fermeture/ouverture C2, C2' qui permet d'autoriser ou non l'alimentation de ladite poche ventricule 50, 50' par la source d'alimentation 40.

Chaque portion de la ligne d'amenée 52 de dialysat au niveau de laquelle est situé un organe amont C2, C2' ou aval C1, C1' de fermeture/ouverture, est souple. Chacun desdits organes C2, C2', C1, C1' de fermeture/ouverture est formé par une pince commandable pour, d'une part, pincer de l'extérieur la paroi de ladite portion souple de la ligne d'amenée 52 de manière à fermer cette portion, et, d'autre part, pour laisser ouverte ladite portion souple de ladite ligne d'amenée 52.

L'utilisation de deux poches ventricules 50, 50' montées en parallèle et alimentables par une source externe, facilite les manipulations et évite d'avoir à ouvrir les enceintes 500, 500' au cours de la dialyse. En effet, au cours d'une séance, les poches ventricules sont laissées dans leur enceinte tandis que leur réapprovisionnement se fait au moyen de la ou des poches nourricières qui sont à l'extérieur des enceintes. La présence des deux poches ventricules et du système de pinçage alternatif formé par les organes C1 à C2' permet de charger successivement et alternativement les poches ventricules à partir de la ou des poches nourricières contenant la réserve. Ceci permet d'alimenter en continu la ligne d'amenée du dialysat à partir de l'une des poches ventricules disposées dans l'enceinte sous pression.

La mise en dépression de l'enceinte d'une poche ventricule 50 ou 50' à l'aide du générateur de vide 80 permet, à l'état ouvert de l'organe amont d'ouverture/fermeture C2 ou C2', et à l'état fermé de l'organe aval d'ouverture/fermeture correspondant C1 ou C1', d'aspirer le dialysat contenu dans la source d'alimentation 40 pour remplir ladite poche ventricule 50 ou 50' (voir figure 5).

Pour commander l'écoulement de dialysat dans la ligne d'amenée 52, l'unité de pilotage 10 commande l'ouverture de l'organe aval C1 ou C1' de l'une des poches ventricules 50 ou 50' et la fermeture de l'organe amont C2 ou C2' correspondant. L'utilisation de deux poches ventricules montées en parallèle permet d'en recharger une pendant que l'autre est en cours d'utilisation.

La ligne 52 d'amenée de dialysat comporte une restriction de passage 520 pour créer une perte de charge et permettre de réguler la pression appliquée à la poche ventricule 50 ou 50' en fonction de la différence de pression mesurée aux bornes de ladite restriction. En particulier, ladite ligne 52 est munie de moyens de mesure de la différence de pression entre l'entrée et la sortie de ladite restriction de passage 520. Cette restriction 520 peut être formée au moins en partie d'un tube calibré de section constante qui forme un étranglement sur une longueur prédéterminée. Ladite restriction de passage 520 est située entre les poches 50, 50' et l'entrée 401 du compartiment dialysat 14.

Lesdits moyens de mise sous pression maintiennent avantageusement une pression sur la poche ventricule 50 ou 50' qui est suffisante pour provoquer l'écoulement du dialysat. La ligne d'amenée est calibrée pour que, pour une pression déterminée appliquée à la poche ventricule 50 ou 50', le débit de dialysat dans ladite ligne ait une valeur sensiblement constante.

Un tel système formé d'un tube à perte de charge et de moyens de mesure de la différence de pression aux bornes de la restriction, permet notamment de déterminer le débit de dialysat qui circule dans ladite ligne d'amenée et entre dans le compartiment dialysat. Grâce au débit de dialysat ainsi mesuré, l'unité de pilotage 10 peut réguler la pression appliquée à la poche ventricule 50 ou 50' pour obtenir le débit de dialysat souhaité entrant dans le compartiment dialysat et maintenir précisément et de manière fiable ce débit à une valeur donnée pendant un temps donné.

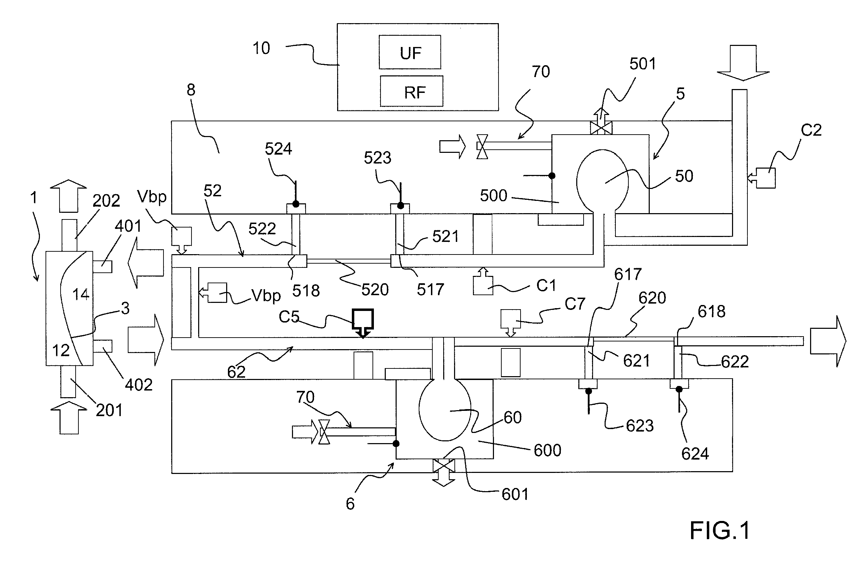

Comme illustré plus particulièrement à la figure 1, lesdits moyens de mesure de la différence de pression aux bornes de la restriction 520 de la ligne d'amenée 52 comprennent deux orifices de prise de pression 517, 518 ménagés, dans la paroi périphérique de ladite ligne 52, l'un en amont et l'autre en aval de ladite restriction 520. Chaque orifice de prise de pression 517, 518 est associé à un capteur de pression 523, 524. Chaque capteur de pression 523, 524 est agencé par rapport à l'orifice de prise de pression correspondant 517, 518 de manière à mesurer la pression dans la ligne d'amenée 52 au niveau dudit orifice de prise de pression 517, 518 tout en étant écarté dudit orifice 517, 518 pour ne pas être en contact avec le dialysat circulant dans la ligne 52, 62.

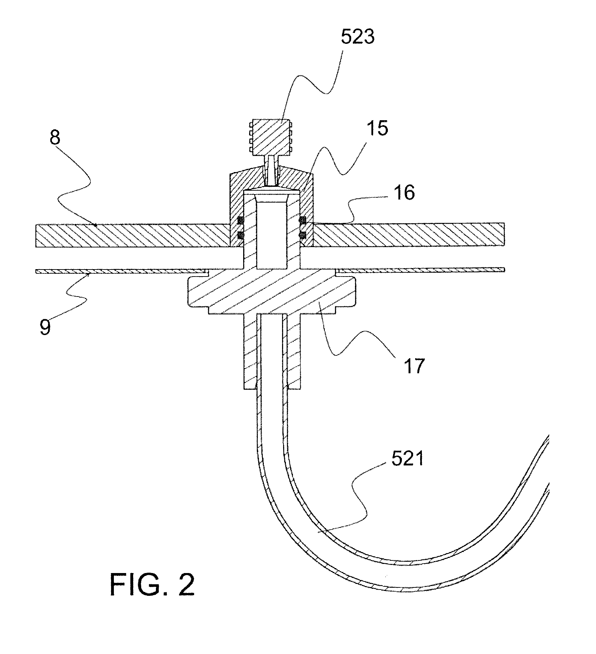

Avantageusement, un conduit 521, 522, muni de préférence d'un filtre 17 (voir figure 2) perméable à l'air et imperméable aux agents infectieux et aux liquides, est interposé entre chaque capteur de pression 523, 524 et l'orifice 517, 518 de prise de pression correspondant qui débouche dans la ligne d'amenée.

Ainsi, comme illustré à la figure 2, le capteur de pression 523 est monté dans une cavité du bâti 8 de la machine à l'aide d'une pièce support 15. Ladite pièce support 15 est creuse et destinée à être couplée au conduit 521 correspondant. Une garniture d'étanchéité 16, telle qu'un joint torique, assure l'étanchéité pneumatique entre le support 15 du capteur de pression et le conduit 521. A l'état inséré d'une cassette 9 dans le bâti, l'extrémité libre du conduit 521, de préférence munie du filtre 17, est engagée dans la pièce support 15 de sorte que le capteur de pression est apte à mesurer la pression dans ledit conduit 521 et ainsi la pression au niveau de la ligne 52 où est ménagé l'orifice de prise de pression correspondant. Le capteur de pression 524 et le conduit de prise de pression 522 associé à l'orifice de prise de pression 518 sont agencés de la même manière que celle décrite à la figure 2 pour le capteur de pression 523.

On peut prévoir que la mesure de pression dans la ligne artérielle L1 et la ligne veineuse L2 soit réalisée de la même manière que pour la prise de pression aux bornes de la restriction 520. On peut ainsi prévoir que le capteur de pression Pa et le capteur de pression Pb soient fixés dans une partie du bâti à écartement d'un orifice de pression ménagé dans la ligne L1, L2 correspondante. Un agencement correspondant à celui illustré à la figure 2 peut alors être utilisé pour permettre aux capteurs de pression Pa et Pv de mesurer la pression dans la ligne L1, L2 correspondante sans risque de contamination.

Ladite machine comprend un système d'ouverture/fermeture de la ligne 62 d'évacuation de dialysat. Ledit système d'ouverture/fermeture autorise, à l'état d'ouverture, la circulation dans ladite ligne d'évacuation 62 du dialysat présent en sortie du compartiment de dialysat 14 pour remplir l'une des poches de décharge 60 ou 60', comme détaillé ci-après, et empêche, à l'état de fermeture, la circulation dans ladite ligne d'évacuation 62 du dialysat présent en sortie dudit compartiment de dialysat 14 de sorte que, pour une pression suffisante dans le compartiment dialysat, ledit dialysat passe à travers la membrane 3 dans le compartiment sang. Ce système d'ouverture/fermeture est détaillé ci-après.

Ledit système d'évacuation du dialysat 6 comprend au moins une poche de décharge 60, de préférence souple, présentant une entrée et une sortie raccordées à la ligne d'évacuation de dialysat 62. Ledit système d'ouverture/fermeture comprend un organe amont d'ouverture/fermeture C5 situé entre la sortie 402 du compartiment dialysat 14 et ladite poche de décharge 60.

Ledit système d'évacuation du dialysat 6 comprend au moins une autre poche de décharge 60' montée en dérivation de ladite poche de décharge 60. Ainsi la poche de décharge 60 et la poche de décharge 60' sont situées sur deux branches de la ligne d'évacuation 62. Autrement dit, en partant de la sortie du compartiment de dialysat 14, la ligne d'évacuation 62 se sépare, en amont des poches de décharge 60, 60', en deux branches parallèle munies l'une de la poche de décharge 60 et l'autre de la poche de décharge 60'. Ces deux branches se raccordent l'une à l'autre en aval des poches 60, 60' et en amont de la restriction de section 620 comme détaillé ci-après.

Ledit système d'ouverture/fermeture comprend un autre organe amont d'ouverture/fermeture C5' situé entre ladite autre poche de décharge 60' et le noeud d'entrée NE6 de raccordement des poches de réserve 60, 60'.

De manière similaire aux organes amont C5, C5', chaque poche de décharge 60, 60' est munie d'un organe aval C6, C6' de fermeture/ouverture situé entre la poche de décharge 60, 60' correspondante et le noeud de sortie NS6 de raccordement des poches de décharge 60, 60'.

Chaque portion de la ligne d'évacuation de dialysat 62 au niveau de laquelle est situé un organe amont C5, C5' ou aval C6, C6' de fermeture/ouverture est souple. Chacun desdits organes amont C5, C5' ou aval C6, C6' de fermeture/ouverture de la ligne d'évacuation de dialysat 62 est formé par une pince commandable pour, d'une part, pincer de l'extérieur la paroi de ladite portion souple de la ligne d'évacuation 62 de manière à fermer cette portion, et, d'autre part, pour laisser ouverte ladite portion souple de ladite ligne d'évacuation 62.

Un tel ensemble d'organes amont C5, C5' et aval C6, C6' de fermeture/ouverture associés aux poches de décharge 60, 60' permet à l'état ouvert de l'organe amont C5, respectivement C5', et fermé de l'organe aval C6, respectivement C6', de remplir la poche de décharge 60, respectivement 60', et, à l'état fermé de l'organe amont C5, respectivement C5', et ouvert de l'organe aval C6, respectivement C6', de vider ladite poche de décharge 60, respectivement 60'.

Dans le cas où le système d'évacuation 6 est formé par une ou plusieurs poches de décharge 60, 60', on considère que la ligne d'évacuation 62 est ouverte lorsque le dialysat peut circuler à travers ladite ligne pour remplir ladite ou l'une desdites poches.

Les moyens d'évacuation du dialysat et du liquide présents dans la poche de décharge 60 ou 60' peuvent être formés par la gravité si la configuration de la poche de décharge s'y prête et/ou par des moyens de mise sous pression 70, de préférence communs à ceux qui servent à la mise sous pression des poches ventricules 50, 50'. Dans ce dernier cas, ladite ou chaque poche 60, 60' est logée dans une enceinte 600, 600' sensiblement étanche apte à être mise sous pression par lesdits moyens de mise sous pression 70. Ladite enceinte présente une sortie 601 dont l'ouverture est contrôlable par une électrovanne pour réduire la pression dans l'enceinte.

On peut aussi prévoir que chaque enceinte 600, 600' qui loge une poche de décharge 60, 60' soit raccordée à un générateur de vide 80. Le générateur de vide 80 et/ou les moyens de mise sous pression 70 permettent d'appliquer une pression ou une dépression à la poche de décharge 60 ou 60', de manière à ajuster la pression moyenne dans le compartiment de dialysat 14 lors d'une phase d'ultrafiltration. Dans ce cas, l'organe aval C6 ou C6' de la poche de décharge 60 ou 60' qui est utilisée pour ajuster la pression moyenne dans le compartiment de dialysat 14, est fermé tandis que l'organe amont C5 ou C5' correspondant est ouvert.

Ledit générateur de vide 80 peut être commun pour la mise en dépression des enceintes du système d'amenée 5 de dialysat et la mise en dépression des enceintes du système d'évacuation 6.

Ladite machine comprend des moyens de détermination de la quantité de dialysat et de liquide récupérée dans la poche de décharge 60 ou 60' qui se vide.

Lesdits moyens de détermination de la quantité de dialysat et de liquide récupérée dans la ou les poches de décharge 60, 60' comprennent une restriction de passage 620, ménagée dans la ligne 62 d'évacuation de dialysat et située en aval desdites poches de décharge 60, 60', et des moyens de mesure de la différence de pression aux bornes de ladite restriction de passage 620.

Lesdits moyens de mesure de la différence de pression aux bornes de la restriction 620 de la ligne d'évacuation 62 sont similaires à ceux associés à la restriction 520 de la ligne d'amenée 52.

Ainsi, lesdits moyens de mesure de la différence de pression aux bornes de la restriction 620 de la ligne d'évacuation 62 comprennent deux orifices de prise de pression 617, 618 ménagés, dans la paroi périphérique de ladite ligne 62, l'un en amont et l'autre en aval de ladite restriction 620.

Chaque orifice de prise de pression 617, 618 est associé à un capteur de pression 623, 624 agencé de manière à mesurer la pression dans la ligne 62 au niveau dudit orifice 617, 618 tout en étant écarté dudit orifice 617, 618 pour ne pas être en contact avec le dialysat circulant dans la ligne 62.

De manière similaire aux moyens de mesure de la différence de pression aux bornes de la restriction 520, un filtre perméable à l'air et imperméable aux agents infectieux et aux liquides, est interposé entre chaque capteur de pression 623, 624 et l'orifice 617, 618 de prise de pression correspondant. En particulier, chaque capteur de pression 623, 624 est monté dans une cavité du bâti 8 de la machine à l'aide d'une pièce support creuse qui est destinée à être couplée au conduit 621, 622 relié à l'orifice de prise de pression 617, 618 correspondant.

De manière similaire à l'agencement illustré à la figure 2 pour le capteur 523, une garniture d'étanchéité, telle qu'un joint torique, assure l'étanchéité pneumatique entre la pièce support, et donc le capteur de pression 623, 624, et le conduit 621, 622. A l'état inséré de la cassette 9 dans le bâti, l'extrémité libre du conduit 621, de préférence munie du filtre, est engagée dans la pièce support de sorte que le capteur de pression est apte à mesurer la pression dans ledit conduit et ainsi la pression au niveau de la ligne 62 où est ménagé l'orifice de prise de pression correspondant.

Les capteurs de pression 623, 624 associés à ladite restriction 620 permettent de déterminer la différence de pression aux bornes de la restriction 620 et ainsi le débit de dialysat et de liquide évacué de la poche de décharge 60 ou 60', ce qui permet de déterminer, en mesurant le temps d'écoulement correspondant, la quantité de dialysat et de liquide récupérée dans ladite poche de décharge 60 ou 60'.

Ladite machine présente, comme expliqué ci-dessus, un bâti 8 et une cassette 9 amovible par rapport au bâti qui est destinée à être insérée dans un logement dudit bâti. Ladite cassette 9 comprend les lignes de dialysat 52, 62 et les poches 50, 50', 60, 60'.

Lesdits capteurs de pression 523, 524, 623, 624 sont fixés au bâti 8 de la machine. A l'état inséré de la cassette 9 dans le bâti 8 de la machine, les conduits de prise de pression sont en communication pneumatique avec les capteurs de pression. Par communication pneumatique on entend que le capteur et le conduit de prise de pression correspondant sont agencés l'un par rapport à l'autre de telle sorte que le capteur est apte à mesurer la pression qui règne dans ledit conduit, et donc la pression qui règne dans la ligne au niveau où est ménagé l'orifice de prise de pression correspondant.

Les lignes d'amenée de dialysat 52 et d'évacuation de dialysat 62 sont aptes à être retirées du reste de la machine, tandis que les capteurs de pression 523, 524, 623, 624 correspondants restent fixés au bâti de la machine.

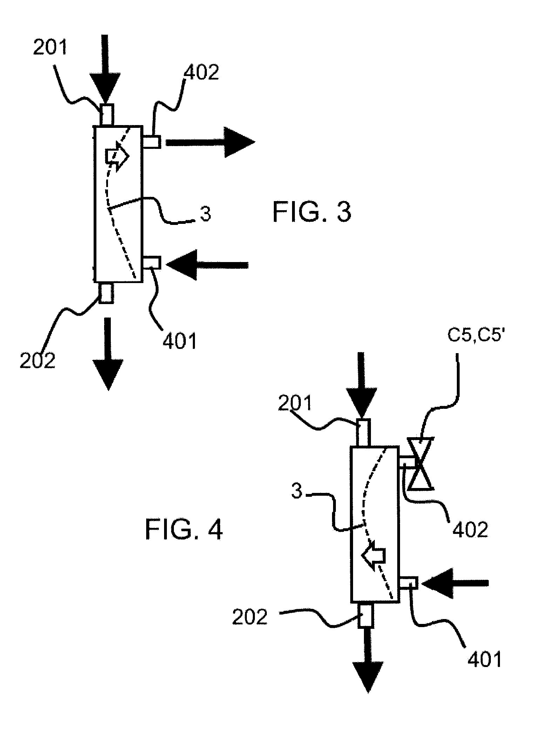

Comme illustré à la figure 4, pour réaliser une étape de rétrofiltration, lesdits moyens de commande de rétrofiltration RF sont configurés pour commander ledit système C5, C5' d'ouverture/fermeture de manière à fermer la ligne 62 d'évacuation, et, à l'état fermé de ladite ligne d'évacuation 62, commander la pression appliquée à la poche ventricule 50 ou 50' pour rétrofiltrer une quantité de dialysat donnée.

En particulier, pour commander la fermeture de la ligne d'évacuation de dialysat 62 en vue d'une étape de rétrofiltration, lesdits moyens de commande de rétrofiltration RF sont configurés pour commander la fermeture de chacun desdits organes amont d'ouverture/fermeture C5, C5'. Parallèlement à cette étape de rétrofiltration, la poche de décharge précédemment remplie peut être vidée en ouvrant l'organe aval C6 ou C6' correspondant.

Pour commander l'ouverture de la ligne d'évacuation de dialysat 62 en vue d'une étape d'ultrafiltration, lesdits moyens de commande d'ultrafiltration UF sont configurés pour commander l'ouverture de l'un desdits organes amont d'ouverture/fermeture, par exemple l'organe C5, et la fermeture de l'autre organe amont d'ouverture/fermeture, par exemple l'organe C5', de sorte que le dialysat en sortie du compartiment 14 soit récupéré par l'une des deux poches de décharge, par exemple la poche 60.

Avantageusement, l'unité de pilotage 10 commande en parallèle la fermeture de l'organe aval de fermeture/ouverture C6 associé à la poche de décharge 60 dont l'organe amont C5 est ouvert, pour permettre à ladite poche de décharge 60 de se remplir.

L'unité de pilotage commande aussi l'ouverture de l'organe aval C6' de fermeture/ouverture associé à la poche de décharge 60' dont l'organe amont C5' est fermé, de manière à vider ladite poche de décharge 60' et mesurer la quantité récupérée dans ladite poche.

Même si l'une des branches de la ligne 62 est fermée, le dialysat qui sort du compartiment de dialysat 14 peut circuler dans ladite ligne 62 par l'autre branche et remplir la poche de décharge correspondante, de sorte que ladite ligne 62 est considérée comme ouverte.

Lesdits moyens de commande d'ultrafiltration UF sont aussi configurés pour, à l'étape d'ultrafiltration suivante, commander l'ouverture de l'autre C5' organe amont d'ouverture/fermeture associé à la poche de décharge 60' et la fermeture dudit organe amont C5 d'ouverture/fermeture associé à la poche de décharge 60. L'unité de pilotage commande en parallèle la fermeture de l'organe aval C6' de fermeture/ouverture associé à la poche de décharge 60' dont l'organe amont C5' est ouvert. Parallèlement, l'unité de pilotage 10 commande l'ouverture de l'organe aval C6 de fermeture/ouverture associé à la poche de décharge 60 dont l'organe amont C5 est fermé, de manière à vider ladite poche et mesurer la quantité récupérée dans ladite poche.

Le vidage d'une poche de décharge peut s'effectuer en temps masqué pendant une étape de rétrofiltration et/ou d'ultrafiltration. La mesure de la quantité de dialysat et de liquide récupérée dans la poche de décharge 60 ou 60', permet d'ajuster la quantité de dialysat à rétrofiltrer pour une étape de rétrofiltration suivante et/ou d'ajuster la quantité aqueuse à ultrafiltrer pour une étape d'ultrafiltration suivante.

L'ultrafiltration permet d'extraire une fraction aqueuse du sang comme illustré à la figure 3. En particulier, dans le cas d'un patient dont les reins ne fonctionnent plus, il n'y a plus de production d'urine, et l'eau issue de son alimentation et de ce qu'il boit n'est plus suffisamment éliminée. Il en résulte alors un surpoids du patient qui peut être dangereux pour sa santé. Pour éliminer l'eau présente en excès dans le sang il est ainsi possible de procéder à l'ultrafiltration du sang.

Pour augmenter l'efficacité du traitement du sang, la machine est aussi conçue pour permettre de retirer du sang une fraction de liquide supérieure à celle qui correspond à l'excès d'eau dans le sang. Dans ce cas, il faut compenser cette extraction importante en réinjectant du dialysat dans le sang pour compenser le volume de liquide extrait en plus de celui qui correspond à l'eau en excès dans le sang. Ces étapes d'ultrafiltration et de rétrofiltration permettent d'améliorer l'épuration du sang du patient tout en conservant son équilibre hydrique.

Ainsi, au début d'une séance de dialyse, l'unité de pilotage 10 détermine à partir des données de poids « sec » et réel saisies, la quantité de fraction aqueuse en excès à retirer du liquide corporel, appelée quantité aqueuse en excès. Le poids "sec" du patient est le poids du patient lorsqu'il ne présente pas d'eau en excès dans son sang.

L'unité de pilotage 10 pilote alors les étapes d'ultrafiltration et de rétrofiltration pour extraire à travers la membrane 3 de dialyse une quantité aqueuse du liquide corporel, appelée quantité aqueuse brute à retirer, supérieure à ladite quantité aqueuse en excès définie, et pour rétrofiltrer une quantité de dialysat sensiblement égale à la différence entre ladite quantité aqueuse brute à retirer et ladite quantité aqueuse en excès. La différence entre ladite quantité aqueuse brute à retirer et ladite quantité aqueuse en excès est aussi appelée quantité ultrafiltrée nette.

Une séance de traitement comprend une pluralité d'étapes d'ultrafiltration en alternance avec des étapes de rétrofiltration. Le nombre d'alternances est de préférence au moins égal à 1000.

La succession d'une étape d'ultrafiltration et d'une étape de rétrofiltration est définie comme formant un cycle. Ladite unité de pilotage exécute ainsi au cours d'une séance de traitement, un nombre donné de cycles, noté N. En particulier, chaque cycle, noté avec l'indice i, i allant de 1 à N, est composé d'une étape d'ultrafiltration de durée Te_uf_i et d'une étape de rétrofiltration de durée Te_rf_i. Comme rappelé ci-dessus, l'indice « i » correspond au cycle i considéré.

Comme expliqué ci-dessus, ladite unité de pilotage 10 détermine la quantité aqueuse en excès dans le liquide corporel, notée Q_aq_exces qui doit être retirée. Puis, pour chaque cycle i, ladite unité de pilotage définit les durées Te_uf_i et Te_rf_i, commande les organes C5, C5', et éventuellement C6, C6', en conséquence, et pilote les moyens de mise sous pression 70 et éventuellement le générateur de vide 80, de telle sorte que :

$$Q\_aq\_exces = \text{Somme sur } i, \text{ pour } i \text{ allant de } 1 \text{ à } N, \text{ de}$$

$$K*[(Pa\_i + Pv\_i)/2 - P14\_uf\_i]*Te\_uf\_i - (Dd\_rf\_i*Te\_rf\_i),$$

K étant un coefficient d'ultrafiltration prédéfini propre à la membrane qui traduit sa capacité d'ultrafiltration,
Pa_i étant la pression en amont du compartiment sang 12 au cours de l'étape d'ultrafiltration du cycle i,
Pv_i la pression en aval du compartiment sang 12 au cours de l'étape d'ultrafiltration du cycle i,
P14_uf_i la pression moyenne dans le compartiment de dialysat 14 au cours de l'étape d'ultrafiltration du cycle i,
Dd_rf_i le débit de dialysat dans le compartiment de dialysat 14 au cours de l'étape de rétrofiltration du cycle i, ledit débit Dd_rf_i étant fonction de la pression appliquée sur la poche ventricule 50 ou 50'.

Pour une quantité Q_aq_exces exprimée en millilitre (ml), les pressions en millimètre de mercure (mmHg), le temps en heure (h), et le débit en ml/h, ledit coefficient K de la membrane de dialyse est exprimé en ml/(h*mmHg). Ledit coefficient K d'une membrane de dialyse est généralement compris entre 50 et 90 ml/(h*mmHg).

Les opérations exécutées par l'unité de pilotage et en particulier par les moyens de commande d'ultrafiltration UF et de rétrofiltration RF sont détaillées ci-dessous.

Après avoir déterminé la quantité aqueuse présente en excès dans le sang, notée Q_aq_exces, l'unité de pilotage détermine une quantité aqueuse à retirer du sang, notée Q_aq_brute, supérieure à Q_aq_exces. Le surplus de quantité aqueuse retirée du sang par rapport à la quantité aqueuse en excès dans le sang Q_aq_exces est destinée à être compensée par rétrofiltration d'une quantité correspondante de dialysat. L'unité de pilotage détermine alors la quantité de fraction aqueuse à retirer du liquide corporel à chaque cycle i, notée Q_aq_brute_i, de sorte que Q_aq_brute = Somme sur i de Q_aq_brute_i.

La perte de charge dans la ligne d'amenée est telle qu'au cours d'une étape d'ultrafiltration, la fraction aqueuse du sang passe plus facilement à travers la membrane sous l'effet de la pression transmembranaire que le dialysat ne passe de la poche ventricule 50 ou 50' vers le dialyseur 1.

Pour exécuter l'étape d'ultrafiltration d'un cycle i, lesdits moyens de commande d'ultrafiltration UF commandent ledit système C5, C5' d'ouverture/fermeture de la ligne d'évacuation de dialysat 62 pour ouvrir ladite ligne d'évacuation de dialysat 62 pendant la durée Te_uf et commandent, à l'état ouvert de la ligne d'évacuation de dialysat 62, la pression, notée P5_uf_i, appliquée à la poche ventricule 50 ou 50' et la pression, ou dépression, à appliquer à la poche de décharge 60 ou 60', pour retirer du sang la quantité aqueuse Q_aq_brute_i.

A cet effet, lesdits moyens d'ultrafiltration déterminent la pression moyenne P14_uf_i, à atteindre dans le compartiment de dialysat 14 et la durée Te_uf_i correspondante qui vérifient la relation Q_aq_brute_i = K*(P14_uf_i-P12_uf_i)* Te_uf_i,

K étant, comme expliqué ci-dessus, un coefficient prédéfini propre à la membrane qui traduit sa capacité d'ultrafiltration,

avec P12_uf_i = (Pa_i+Pv_i)/2, c'est-à-dire la pression moyenne dans le compartiment sang 12,

où Pa_i est la pression en amont du compartiment sang 12 au cours de l'étape d'ultrafiltration du cycle i, et
où Pv_i est la pression en aval du compartiment sang 12 au cours de l'étape d'ultrafiltration du cycle i,

Pendant ladite durée Te_uf_i, lesdits moyens de commande d'ultrafiltration UF commandent la pression P5_uf_i à appliquer à ladite poche ventricule 50 ou 50' et la pression, ou dépression, à appliquer à la poche de décharge 60 ou 60', permettant d'obtenir ladite pression P14_uf_i dans le compartiment de dialysat 14.

Avantageusement, ladite pression P5_uf_i est régulée à l'aide des capteurs de pression 523, 524 associés à la restriction 520 pour obtenir un débit fixe de dialysat quelle que soit la pression moyenne dans le compartiment de dialysat.

Pour commander l'étape de rétrofiltration d'un cycle i, lesdits moyens de commande de rétrofiltration RF commandent ledit système C5, C5' d'ouverture/fermeture de la ligne d'évacuation de dialysat 62 pour fermer ladite ligne d'évacuation de dialysat 62 pendant la durée Te_rf_i. Puis, pendant cette durée Te_rf_i, lesdits moyens de commande de rétrofiltration RF commandent la pression, notée P5_rf_i, appliquée à ladite poche ventricule 50 ou 50' pour rétrofiltrer une quantité de dialysat donnée Q_rf_i.

Préférentiellement, ladite quantité de dialysat à rétrofiltrer Q_rf_i est définie comme étant égale à la quantité de dialysat et de liquide qui a été récupérée dans la poche de décharge 60 ou 60' lors d'une étape d'ultrafiltration précédente, moins la quantité de dialysat écoulée de la poche ventricule 50 ou 50' au cours de ladite étape d'ultrafiltration précédente.

Ladite quantité de dialysat rétrofiltrée Q_rf_i est égale à Dd_rf_i*Te_rf_i, Dd_rf_i étant le débit de dialysat dans la ligne d'amenée 52 qui entre dans le compartiment dialysat 14. Lesdits moyens de commande de rétrofiltration RF commandent alors la pression P5_rf_i appliquée à la poche ventricule 50 ou 50' de manière à obtenir le débit de dialysat Dd_rf_i souhaité. Le débit de dialysat Dd_rf_i réel qui traverse la ligne d'amenée 52 est calculé à l'aide de la différence de pression mesurée aux bornes de la restriction 520, et l'unité de pilotage régule P5_rf_i pour maintenir le débit de dialysat Dd_rf_i à la valeur souhaitée.

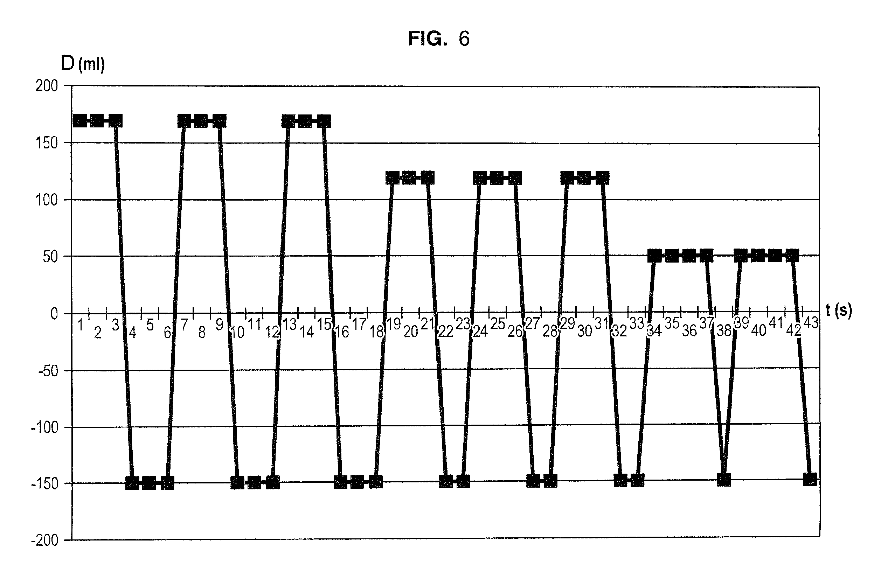

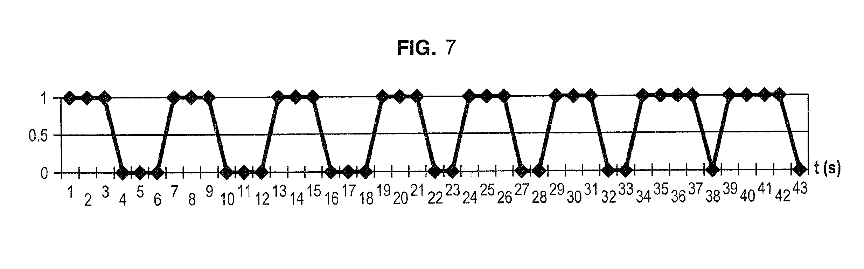

Un exemple de traitement est illustré aux figures 6 et 7 pour une durée de 43s. La figure 6 donne, en fonction du temps t(s), le débit D (ml) transmembranaire qui résulte d'une pluralité de cycles d'ultrafiltration et de rétrofiltration. Dans cet exemple, au cours d'une première étape d'ultrafiltration de 3 secondes, le débit d'ultrafiltration vaut 170 ml/min. Cette étape d'ultrafiltration est suivie d'une étape de rétrofiltration de 3 secondes aussi, qui se traduit par un débit négatif de 150 ml/min. Ces deux étapes forment un cycle qui est répété deux fois. Le quatrième cycle, répété aussi deux fois, comprend une étape d'ultrafiltration de même durée, mais de plus faible débit par rapport aux trois cycles précédents. Les étapes correspondantes de rétrofiltration présentent un même débit mais une durée plus courte par rapport aux trois cycles précédents. Enfin, le septième cycle, qui est répété une fois, comprend une étape d'ultrafiltration de plus grande durée que celle des autres cycles mais de plus faible débit, tandis que l'étape de rétrofiltration correspondante est encore réalisée avec un même débit mais sur une durée plus courte que celle des autres cycles. Il est ainsi possible d'ajuster pour chaque étape d'ultrafiltration et de rétrofiltration le débit correspondant et la durée de l'étape. La figure 7 montre la configuration ouverte (1) ou fermée (0) de la ligne d'évacuation au cours de la pluralité de cycles d'ultrafiltration et de rétrofiltration de la figure 6.

On notera que chacun de ces trois groupes de cycles permet d'obtenir une même quantité ultrafiltrée nette (ou globale) à partir de quantités rétrofiltrées différentes en ajustant la pression appliquée à la poche de décharge 60 ou 60' au cours des opérations d'ultrafiltration et/ou leur durée.

La machine de dialyse selon l'invention est aussi munie de composants classiques pour assurer un traitement fiable et efficace du liquide corporel à traiter, notamment un détecteur de fuite de sang FS ménagé dans la ligne d'évacuation 62. Avantageusement, l'unité de pilotage est configurée pour permettre d'étalonner les débitmètres l'un par rapport à l'autre.

Pour une meilleure lisibilité des figures, les connexions entre l'unité de pilotage 10 et les différents composants de la machine avec lesquels ladite unité de pilotage 10 communique pour les commander et/ou récupérer des données, n'ont pas été représentées. Ces composants sont notamment les organes C1 à C6', CV, les électrovannes, les capteurs de pression, les capteurs de température ainsi que les moyens de mise sous pression 70, le générateur de vide 80, la pompe à sang Psg et le système d'injection PH, et le détecteur d'air DA ou de fuite de sang FS.

Chaque enceinte 500, 500' est munie de moyens de chauffage ou préchauffage de l'enceinte. Chaque enceinte 500, 500', 600, 600' est aussi munie d'une vanne de mise à l'air V2, V5, V8, V11 associée à un filtre à air F1, F2, F3, F4, et d'un capteur de pression P7, P8, P9 et P10.

Les moyens de mise sous pression 70 comprennent les éléments suivants : un réservoir d'air comprimé R1, un capteur de pression P11 du réservoir R1, un compresseur Pa1, un clapet anti-retour pour l'air comprimé Ar1, et un filtre à air et silencieux pour l'air comprimé Si1.

Le générateur de vide 80 comprend un réservoir de vide d'air R2, un capteur de pression P12 du réservoir R2, une pompe à vide Pa2, un clapet anti-retour pour le vide d'air Ar2 et un filtre à air et silencieux pour le vide d'air Si 2.

La ligne d'amenée 52 est aussi munie de moyens de chauffage Ch3 du dialysat associés à des moyens de mesure de température T1, T2, T3. La ligne d'évacuation 62 est munie de moyens de mesure de température T4, T5 en aval des poches 60, 60'.

Avantageusement, la ligne veineuse L2 comprend aussi un organe de fermeture/ouverture CV, tel qu'une pince.

La présente invention n'est nullement limitée aux modes de réalisation décrits et représentés, mais l'homme du métier saura y apporter toute variante conforme à l'invention telle que définie dans les revendications.

Une telle solution permet de s'exempter de lignes de sous-tirage et de réinjection spécifiques comme il en existe dans les solutions connues de l'état de la technique.

En outre, la machine selon l'invention ne nécessite qu'un seul filtre dialyseur pour chaque séance. Il n'est pas nécessaire de prévoir deux filtres en série comme il en existe dans les solutions connues de l'état de la technique.

La machine comprend au moins une ligne de by-pass L10, ainsi qu'un ou plusieurs organes Vbp de fermeture/ouverture associés. La ligne de by-pass L10 est utilisée pour réaliser l'amorçage et la calibration du circuit de dialysat, et pour permettre de ne pas injecter dans le dialyseur du dialysat défectueux, par exemple du fait d'un problème de température.

Avantageusement, la ligne d'évacuation du dialyseur est raccordée à un récipient de récupération ou à un système de mise à l'égout Egt.

## Revendications

1. Machine de dialyse permettant de traiter un liquide corporel, tel que du sang ou du plasma, ladite machine comportant :

    - un dialyseur (1) comprenant une enceinte logeant une membrane (3) de dialyse qui sépare l'enceinte en un compartiment de passage dudit liquide corporel, appelé compartiment de liquide corporel (12), et en un compartiment de passage de dialysat, appelé compartiment de dialysat (14),

    - ledit compartiment de liquide corporel (12), respectivement ledit compartiment de dialysat (14), présentant une entrée (201) de liquide corporel, respectivement une entrée de dialysat (401), et une sortie (202) de liquide corporel, respectivement une sortie de dialysat (402),

    - un système d'amenée du dialysat (5) comprenant une ligne d'amenée (52) de dialysat raccordée à l'entrée (401) du compartiment de dialysat (14), et

    - un système d'évacuation du dialysat (6) comprenant une ligne d'évacuation (62) de dialysat raccordée à la sortie (402) du compartiment de dialysat (14),

    - une unité de pilotage (10), telle qu'un automate programmable, qui comprend :

        - des moyens de commande d'ultrafiltration (UF) permettant de faire passer, à travers au moins une partie de la membrane (3), une quantité aqueuse du liquide corporel depuis le compartiment de liquide corporel (12) jusque dans le compartiment de dialysat (14), et

        - des moyens de commande de rétrofiltration (RF) permettant de faire passer le dialysat, à travers au moins une partie de la membrane (3), depuis le compartiment de dialysat (14) jusque dans le compartiment de liquide corporel (12),

    **caractérisée en ce que**, la ligne (52) d'amenée de dialysat comportant une restriction de passage (520), ladite machine comprend des moyens de mesure de la différence de pression (523, 524) aux bornes de ladite restriction de passage (520),

    **en ce que** ledit système d'amenée du dialysat (5) comprend aussi :

        - au moins une poche (50) souple, appelée poche ventricule, destinée à contenir du dialysat et raccordée à ladite ligne d'amenée (52) en amont de ladite restriction de passage (520), ladite poche ventricule (50) étant contenue dans une enceinte (500), et
- des moyens de mise sous pression de la poche ventricule (50) par pressurisation de ladite enceinte (500), et **en ce que** ladite machine comprend un système (C5, C5') d'ouverture/fermeture de la ligne (62) d'évacuation de dialysat autorisant, à l'état d'ouverture, la circulation dans ladite ligne d'évacuation (62) du dialysat présent en sortie du compartiment de dialysat (14) et empêchant, à l'état de fermeture, la circulation dans ladite ligne d'évacuation (62) du dialysat présent en sortie dudit compartiment de dialysat (14),

lesdits moyens de commande de rétrofiltration (RF) étant configurés pour :
- commander ledit système (C5, C5') d'ouverture/fermeture pour fermer la ligne (62) d'évacuation, et
- à l'état fermé de ladite ligne d'évacuation (62), commander la pression appliquée à ladite poche ventricule (50), en fonction de la différence de pression mesurée aux bornes de la restriction (520), pour obtenir un débit de dialysat donné.

2. Machine selon la revendication 1, **caractérisée en ce que** ledit système d'évacuation du dialysat (6) comprend au moins une poche de décharge (60), de préférence souple, présentant une entrée et une sortie raccordées à la ligne d'évacuation de dialysat (62),
et **en ce que** ledit système (C5, C5') d'ouverture/fermeture comprend un organe amont d'ouverture/fermeture (C5) situé entre la sortie (402) du compartiment dialysat (14) et ladite poche de décharge (60),
et **en ce que** ladite machine comprend des moyens de détermination de la quantité de dialysat et de liquide récupérée dans ladite poche de décharge (60).

3. Machine selon la revendication 2, **caractérisée en ce que** ledit système d'évacuation du dialysat (6) comprend au moins une autre poche de décharge (60') montée en dérivation de ladite poche de décharge (60),
et **en ce que** ledit système (C5, C5') d'ouverture/fermeture comprend un autre organe amont d'ouverture/fermeture (C5') situé entre ladite autre poche de décharge (60') et le noeud d'entrée (NE6) de raccordement des poches de décharge (60, 60'),
et **en ce que** ladite machine comprend des moyens de détermination de la quantité de dialysat et de liquide récupérée dans ladite autre poche de décharge (60').

4. Machine selon la revendication 2 ou 3, **caractérisée en ce que** lesdits moyens de détermination de la quantité de dialysat et de liquide récupérée dans la ou les poches de décharge (60) comprennent une restriction de passage (620) ménagée dans la ligne (62) d'évacuation de dialysat et située en aval de la ou desdites poches de décharge (60, 60'), et des moyens de mesure de la différence de pression aux bornes de ladite restriction de passage (620).

5. Machine selon l'une des revendications précédentes, **caractérisée en ce que** lesdits moyens de mesure de la différence de pression aux bornes de la restriction (520) de la ligne d'amenée (52), respectivement aux bornes de la restriction (620) de la ligne d'évacuation (62) lorsque ladite restriction est présente, comprennent :

- deux orifices (517, 518; 617, 618), dits orifices de prise de pression, ménagés l'un en amont et l'autre en aval de ladite restriction (520 ; 620), et,
- pour chaque orifice de prise de pression (517, 518 ; 617, 618), un capteur de pression (523, 524 ; 623, 624) agencé de manière à mesurer la pression dans la ligne (52, 62) correspondante au niveau dudit orifice (517, 518; 617, 618) tout en étant écarté dudit orifice (517, 518 ; 617, 618) pour ne pas être en contact avec le dialysat circulant dans la ligne (52, 62).

6. Machine selon la revendication 5, **caractérisée en ce qu'**un filtre (17) perméable à l'air et imperméable aux agents infectieux et aux liquides, est interposé entre chaque capteur de pression (523, 524 ; 623, 624) et l'orifice (517, 518 ; 617, 618) de prise de pression correspondant.

7. Machine selon l'une des revendications 5 et 6, **caractérisée en ce que**, ladite machine présentant un bâti (8), lesdits capteurs de pression (523, 524, 623, 624) sont fixés au bâti de la machine,
et **en ce que** les lignes d'amenée de dialysat (52) et d'évacuation de dialysat (62) sont aptes à être retirées du reste de la machine, tandis que les capteurs de pression (523, 524, 623, 624) correspondants restent fixés au bâti de la machine.

8. Machine selon l'une des revendications 4 à 7 prise en combinaison de la revendication 3, **caractérisée en ce que**, pour commander la fermeture de la ligne d'évacuation de dialysat (62) en vue d'une étape de rétrofiltration, lesdits moyens de commande de rétrofiltration (RF) sont configurés pour commander la fermeture de chacun desdits organes amont d'ouverture/fermeture (C5, C5') associés aux poches de décharge (60, 60'),

et **en ce que**, pour commander l'ouverture de la ligne d'évacuation de dialysat (62) en vue d'une étape d'ultrafiltration, lesdits moyens de commande d'ultrafiltration (UF) sont configurés pour commander l'ouverture de l'un (C5) desdits organes amont d'ouverture/fermeture (C5, C5') et la fermeture de l'autre (C5') organe amont d'ouverture/fermeture, lesdits moyens de commande d'ultrafiltration étant de préférence configurés pour, à l'étape d'ultrafiltration suivante, commander l'ouverture de l'autre (C5') desdits organes amont d'ouverture/fermeture (C5, C5') et la fermeture dudit (C5) organe amont d'ouverture/fermeture.

**9.** Machine selon l'une des revendications 3 à 8 prise en combinaison de la revendication 2, **caractérisée en ce que** la ou chaque poche de décharge (60, 60') est munie d'un organe, dit aval, d'ouverture/fermeture (C6, C6') situé en aval de ladite poche de décharge (60, 60'),

et **en ce que**, après une étape d'ultrafiltration, ladite unité de pilotage est configurée pour commander, de préférence en parallèle d'une étape de rétrofiltration, l'ouverture de l'organe aval d'ouverture/fermeture (C6, C6') de la poche de décharge (60, 60') précédemment remplie afin d'évacuer son contenu,

et, de préférence, pour déterminer la quantité récupérée dans ladite poche de décharge (60, 60'), l'unité de pilotage (10) est configurée pour mesurer le débit qui s'écoule de ladite poche de décharge (60, 60'), à l'aide desdits moyens de mesure de la différence de pression aux bornes de la restriction (620) située en aval de ladite poche de décharge (60, 60').

**10.** Machine selon l'une des revendications précédentes, **caractérisée en ce que** ladite unité de pilotage comprend aussi des moyens de détermination d'une quantité aqueuse en excès à retirer du liquide corporel, appelée quantité aqueuse en excès,

et **en ce que** les moyens de commande d'ultrafiltration (UF) sont configurés pour extraire à travers la membrane (3) de dialyse une quantité aqueuse du liquide corporel, appelée quantité aqueuse brute à retirer, supérieure à ladite quantité aqueuse en excès définie,

les moyens de commande de rétrofiltration (RF) étant configurés pour rétrofiltrer une quantité de dialysat sensiblement égale à la différence entre ladite quantité aqueuse brute à retirer et ladite quantité aqueuse en excès.

**11.** Machine selon l'une des revendications précédentes, **caractérisée en ce que** ladite unité de pilotage (10) est configurée pour commander au cours d'une séance de traitement des étapes d'ultrafiltration en alternance avec des étapes de rétrofiltration, le nombre d'alternances étant de préférence au moins égal à 1000.

**12.** Machine selon la revendication 11, **caractérisée en ce que**, pour un nombre, noté N, de cycles, chaque cycle i, i allant de 1 à N, étant composé d'une étape d'ultrafiltration de durée Te_uf_i et d'une étape de rétrofiltration de durée Te_rf_i,

ladite unité de pilotage (10) est configurée pour :

- déterminer la quantité aqueuse en excès dans le liquide corporel, notée Q_aq_exces qui doit être retirée;
- pour chaque cycle i, définir les durées Te_uf_i et Te_rf_i, et piloter les moyens de mise sous pression (70) de telle sorte que :

$$Q\_aq\_exces = \text{Somme sur } i, \text{ pour } i \text{ allant de 1 à } N, \text{ de}$$

$$K*[(Pa\_i + Pv\_i)/2 - P14\_uf\_i]*Te\_uf\_i - (Dd\_rf\_i*Te\_rf\_i),$$

K étant un coefficient d'ultrafiltration prédéfini propre à la membrane qui traduit sa capacité d'ultrafiltration, Pa_i étant la pression en amont du compartiment de liquide corporel (12) au cours de l'étape d'ultrafiltration du cycle i,

Pv_i la pression en aval du compartiment de liquide corporel (12) au cours de l'étape d'ultrafiltration du cycle i, P14_uf_i la pression moyenne dans le compartiment de dialysat (14) au cours de l'étape d'ultrafiltration du cycle i,

Dd_rf_i le débit de dialysat dans le compartiment de dialysat (14) au cours de l'étape de rétrofiltration du cycle i.

**13.** Machine selon l'une des revendications précédentes, **caractérisé en ce que** ledit système (C5, C5') d'ouverture/fermeture de la ligne d'évacuation (62) comprend au moins une pince apte, d'une part, à pincer de l'extérieur la paroi d'une portion souple de ladite ligne d'évacuation (62) de manière à fermer cette portion, et, d'autre part, à laisser ouverte ladite portion souple de ladite ligne d'évacuation (62).

14. Machine selon l'une des revendications 4 à 12 prise en combinaison de la revendication 3, **caractérisée en ce que** chacun des organes amont (C5, C5'), et, le cas échéant, aval (C6, C6'), de fermeture/ouverture de la ligne d'évacuation (62) comprend au moins une pince apte, d'une part, à pincer de l'extérieur la paroi d'une portion souple de ladite ligne d'évacuation (62) de manière à fermer cette portion, et, d'autre part, à laisser ouverte ladite portion souple de ladite ligne d'évacuation (62).

15. Machine selon l'une des revendications précédentes, **caractérisée en ce que** le système d'amenée (5) du dialysat comprend une poche ventricule supplémentaire (50'), les deux poches ventricules (50, 50') étant aptes à être mises sous pression séparément par lesdits moyens de mise sous pression (70).

16. Machine selon l'une des revendications précédentes, **caractérisée en ce que** ladite ou chaque poche ventricule (50, 50') comprend aussi une entrée de raccordement à une source d'alimentation (40) en dialysat, de préférence une poche nourricière de plus grand volume que la ou chaque poche ventricule (50, 50').

**Patentansprüche**

1. Dialysemaschine, die erlaubt, eine Körperflüssigkeit wie Blut oder Plasma zu behandeln, wobei die Maschine aufweist:

- einen Dialysator (1), der einen Raum aufweist, der eine Dialysemembran (3) aufnimmt, die den Raum in ein Abteil für den Durchgang der Körperflüssigkeit, als Körperflüssigkeitsabteil (12) bezeichnet, und in ein Abteil für den Durchgang des Dialysats, als Dialysatabteil (14) bezeichnet, trennt,
- wobei das Körperflüssigkeitsabteil (12) beziehungsweise das Dialysatabteil (14) einen Körperflüssigkeitseingang (201) beziehungsweise einen Dialysateingang (401) und einen Körperflüssigkeitsausgang (202) beziehungsweise einen Dialysatausgang (402) aufweist,
- ein Dialysat-Zuführsystem (5), das eine Dialysat-Zufuhrleitung (52) umfasst, die mit dem Eingang (401) des Dialysatabteils (14) verbunden ist, und
- ein Dialysat-Ableitungssystem (6), das eine Dialysat-Ableitungsleitung (62) umfasst, die mit dem Ausgang (402) des Dialysatabteils (14) verbunden ist,
- eine Steuereinheit (10) wie ein programmierbarer Automat, der umfasst:

- Ultrafiltrations-Steuermittel (UF), die erlauben, dass durch mindestens einen Teil der Membran (3) eine wässrige Menge der Körperflüssigkeit vom Körperflüssigkeitsabteil (12) bis in das Dialysatabteil (14) strömt, und
- Rückfiltrierungs-Steuermittel (RF), die erlauben, dass das Dialysat durch mindestens einen Teil de la Membran (3) vom Dialysatabteil (14) bis in das Körperflüssigkeitsabteil (12) strömt,

**dadurch gekennzeichnet, dass**, wobei die Dialysat-Zufuhrleitung (52) eine Durchgangsbeschränkung (520) aufweist, die Maschine Messmittel der Druckdifferenz (523, 524) an den Klemmen der Durchgangsbeschränkung (520) umfasst,
dass das Dialysat-Zuführsystem (5) ebenfalls umfasst:

- mindestens einen elastischen Beutel (50), als Ventrikelbeutel bezeichnet, der bestimmt ist, Dialysat zu enthalten und mit der Zufuhrleitung (52) vor der Durchgangsbeschränkung (520) verbunden ist, wobei der Ventrikelbeutel (50) in einem Raum (500) enthalten ist, und
- Mittel für die Unterdrucksetzung des Ventrikelbeutels (50) durch Druckbeaufschlagung des Raums (500),

und dass die Maschine ein Öffnungs-/Schließ-System (C5, C5') der Dialysat-Ableitungsleitung (62) umfasst, das in geöffnetem Zustand die Zirkulation in der Ableitungsleitung (62) des am Ausgang des Dialysatabteils (14) vorhandenen Dialysats erlaubt und in geschlossenem Zustand die Zirkulation in der Ableitungsleitung (62) des am Ausgang des Dialysatabteils (14) vorhandenen Dialysats verhindert,
wobei die Rückfiltrierungs-Steuermittel (RF) konfiguriert sind, um:

- das Öffnungs-/Schließ-System (C5, C5') zu steuern, um die Ableitungsleitung (62) zu schließen, und
- in geschlossenem Zustand der Ableitungsleitung (62) den auf den Ventrikelbeutel (50) in Abhängigkeit von

18

der an den Klemmen der Beschränkung (520) gemessenen Druckdifferenz angewendeten Druck zu steuern, um einen bestimmten Dialysatdurchsatz zu erhalten.

2. Maschine nach Anspruch 1, **dadurch gekennzeichnet, dass** das Dialysat-Ableitungssystem (6) mindestens einen vorzugsweise elastischen Entlastungsbeutel (60) umfasst, der einen Eingang und einen Ausgang aufweist, die mit der Dialysat-Ableitungsleitung (62) verbunden sind,
und dass das Öffnungs-/Schließ-System (C5, C5') ein oberstromiges Öffnungs-/Schließ-Organ (C5) umfasst, das sich zwischen dem Ausgang (402) des Dialysatabteils (14) und dem Entlastungsbeutel (60) befindet,
und dass die Maschine Bestimmungsmittel der in dem Entlastungsbeutel (60) aufgenommen Menge des Dialysats und der Flüssigkeit umfasst.

3. Maschine nach Anspruch 2, **dadurch gekennzeichnet, dass** das Dialysat-Ableitungssystem (6) mindestens einen anderen Entlastungsbeutel (60') umfasst, der in Umleitung von dem Entlastungsbeutel (60) montiert ist,
und dass das Öffnungs-/Schließ-System (C5, C5') ein anderes oberstromiges Öffnungs-/Schließ-Organ (C5') umfasst, das sich zwischen dem anderen Entlastungsbeutel (60') und dem Eingangs-Verbindungsknoten (NE6) der Entlastungsbeutel (60, 60') befindet,
und dass die Maschine Bestimmungsmittel der in dem anderen Entlastungsbeutel (60') aufgenommen Menge des Dialysats und der Flüssigkeit umfasst.

4. Maschine nach Anspruch 2 oder 3, **dadurch gekennzeichnet, dass** die Bestimmungsmittel der in dem oder den Entlastungsbeuteln (60) aufgenommenen Menge des Dialysats und der Flüssigkeit eine Durchgangsbeschränkung (620), die in die Dialysat-Ableitungsleitung (62) eingearbeitet ist und sich unterstromig von der oder den Entlastungsbeuteln (60, 60') befindet, und Messmittel der Druckdifferenz an den Klemmen der Durchgangsbeschränkung (620) umfassen.

5. Maschine nach einem der vorangehenden Ansprüche, **dadurch gekennzeichnet, dass** die Messmittel der Druckdifferenz an den Klemmen der Beschränkung (520) der Zufuhrleitung (52) beziehungsweise an den Klemmen der Beschränkung (620) der Ableitungsleitung (62), wenn die Beschränkung vorhanden ist, umfassen:

    - zwei Öffnungen (517, 518; 617, 618), als Druckmessöffnungen bezeichnet, von denen eine oberstromig und die andere unterstromig von der Beschränkung (520; 620) ausgebildet ist, und,
    - für jede Druckmessöffnung (517, 518; 617, 618), einen Drucksensor (523, 524; 623, 624), der derart ausgebildet ist, dass der Druck in der entsprechenden Leitung (52, 62) im Bereich der Öffnung (517, 518; 617, 618) gemessen wird, wobei er dabei von der Öffnung (517, 518; 617, 618) beabstandet ist, um nicht mit dem in der Leitung (52, 62) zirkulierenden Dialysat im Kontakt zu sein.

6. Maschine nach Anspruch 5, **dadurch gekennzeichnet, dass** ein Filter (17), der luftdurchlässig und für infektiöse Substanzen und für Flüssigkeiten undurchlässig ist, zwischen jedem Drucksensor (523, 524; 623, 624) und der entsprechenden Druckmessöffnung (517, 518; 617, 618) angeordnet ist.

7. Maschine nach einem der Ansprüche 5 und 6, **dadurch gekennzeichnet, dass**, wobei die Maschine ein Gestell (8) aufweist, die Drucksensoren (523, 524, 623, 624) auf dem Gestell der Maschine befestigt sind,
und dass die Dialysat-Zufuhr- (52) und Dialysat-Ableitungsleitungen (62) imstande sind, vom Rest der Maschine entfernt zu werden, wogegen die entsprechenden Durcksensoren (523, 524, 623, 624) auf dem Gestell der Maschine befestigt bleiben.

8. Maschine nach einem der Ansprüche 4 bis 7, herangezogen in Kombination mit Anspruch 3, **dadurch gekennzeichnet, dass**, um das Schließen der Dialysat-Ableitungsleitung (62) zwecks eines Rückfiltrierungsschritts zu steuern, die Rückfiltrierungs-Steuermittel (RF) konfiguriert sind, um das Schließen jedes der den Entlastungsbeuteln (60, 60') zugeordneten oberstromigen Öffnungs-/Schließ-Organe (C5, C5') zu steuern,
und dass, um das Öffnen der Dialysat-Ableitungsleitung (62) zwecks eines Ultrafiltrationsschritts zu steuern, die Ultrafiltrationssteuermittel (UF) konfiguriert sind, um das Öffnen eines (C5) der oberstromiges Öffnungs-/Schließ-Organe (C5, C5') und das Schließen des anderen oberstromigen Öffnungs-/Schließ-Organs (C5') zu steuern,
wobei die Ultrafiltrationssteuermittel vorzugsweise konfiguriert sind, um beim folgenden Ultrafiltrationsschritt das Öffnen des anderen (C5') der oberstromigen Öffnungs-/Schließ-Organe (C5, C5') und das Schließen des oberstromigen Öffnungs-/Schließ-Organs (C5) zu steuern.

9. Maschine nach einem der Ansprüche 3 bis 8, herangezogen in Kombination mit Anspruch 2, **dadurch gekenn-**

**zeichnet, dass** der oder jeder Entlastungsbeutel (60, 60') mit einem unterstromigen Öffnungs-/Schließ-Organ (C6, C6') ausgestattet ist, das sich unterstromig vom Entlastungsbeutel (60, 60') befindet,

und dass, nach einem Ultrafiltrationsschritts, die Steuereinheit konfiguriert ist, um, vorzugsweise parallel mit einem Rückfiltrierungsschritt, das Öffnen des unterstromigen Öffnungs-/Schließ-Organs (C6, C6') des zuvor gefüllten Entlastungsbeutels (60, 60') zu steuern, um seinen Inhalt zu entleeren,

und, vorzugsweise, um die in dem Entlastungsbeutel (60, 60') aufgenommene Menge zu bestimmen, die Steuereinheit (10) konfiguriert ist, um den Durchsatz, der aus dem Entlastungsbeutel (60, 60') abfließt, mit Hilfe der Messmittel der Druckdifferenz an den Klemmen der Beschränkung (620), die sich unterstromig von dem Entlastungsbeutel (60, 60') befindet, zu messen.

10. Maschine nach einem der vorangehenden Ansprüche, **dadurch gekennzeichnet, dass** die Steuereinheit auch Bestimmungsmittel einer überschüssigen wässrige Menge umfasst, die aus der Körperflüssigkeit zu entfernen ist, als überschüssige wässrige Menge bezeichnet,

und dass die Ultrafiltrationssteuermittel (UF) konfiguriert sind, um durch die Dialysemembran (3) eine wässrige Menge der Körperflüssigkeit zu extrahieren, als zu entfernende wässrige Bruttomenge bezeichnet, die größer als die bestimmte überschüssige wässrige Menge ist,

wobei die Rückfiltrierungs-Steuermittel (RF) konfiguriert sind, um eine Dialysatmenge rückzufiltern, die etwa der Differenz zwischen der zu entfernenden wässrigen Bruttomenge und der überschüssigen wässrigen Menge entspricht.

11. Maschine nach einem der vorangehenden Ansprüche, **dadurch gekennzeichnet, dass** die Steuereinheit (10) konfiguriert ist, um während einer Behandlungssitzung Ultrafiltrationsschritte abwechselnd mit Rückfiltrierungsschritten zu steuern, wobei die Anzahl der Wechsel vorzugsweise mindestens gleich 1000 ist.

12. Maschine nach Anspruch 11, **dadurch gekennzeichnet, dass** für eine Anzahl von N Zyklen jeder Zyklus i, wobei i 1 bis N ist, aus einem Ultrafiltrationsschritts der Dauer Te_uf_i und einem Rückfiltrierungsschritts der Dauer Te_rf_i zusammengesetzt ist, wobei die Steuereinheit (10) konfiguriert ist, um:

- die in der Körperflüssigkeit überschüssige wässrige Menge Q_aq_exces zu bestimmen, die entfernt werden muss,
- für jeden Zyklus i die Zeiten Te_uf_i und Te_rf_i zu bestimmen und die Druckbeaufschlagungsmittel (70) derart zu steuern, dass:

$$Q\_aq\_exces = \text{Summe über i, für i von 1 bis N, von}$$
$$K^*[(Pa\_i + Pv\_i)/2 - P14\_uf\_i]^*Te\_uf\_i - (Dd\_rf\_i^*Te\_rf\_i),$$

wobei K ein membraneigener vorbestimmter Ultrafiltierungskoeffizient ist, der ihre Ultrafiltrationsfähigkeit darstellt,

wobei Pa_i der oberstromige Druck des Körperflüssigkeitsabteils (12) während des Ultrafiltrationsschritts des Zyklus i ist,

wobei Pv_i der unterstromige Druck des Körperflüssigkeitsabteils (12) während des Ultrafiltrationsschritts des Zyklus i ist,

wobei P14_uf_i der mittlere Druck im Dialysatabteil (14) während des Ultrafiltrationsschritts des Zyklus i ist,

wobei Dd_rf_i der Dialysatdurchsatz im Dialysatabteil (14) während des Rückfiltrierungsschritts des Zyklus i ist.

13. Maschine nach einem der vorangehenden Ansprüche, **dadurch gekennzeichnet, dass** das Öffnungs-/Schließ-System (C5, C5') der Ableitungsleitung (62) mindestens eine Klammer umfasst, die imstande ist, einerseits von außerhalb der Wand einen elastischen Abschnitt der Ableitungsleitung (62) zu klammern, um diesen Abschnitt zu schließen, und andererseits den elastischen Abschnitt der Ableitungsleitung (62) offen zu lassen.

14. Maschine nach einem der Ansprüche 4 bis 12, herangezogen in Kombination mit Anspruch 3, **dadurch gekennzeichnet, dass** jedes der oberstromigen (C5, C5') und gegebenenfalls unterstromigen Öffnungs-/Schließ-Organe (C6, C6') der Ableitungsleitung (62) mindestens eine Klammer umfasst, die imstande ist, einerseits von außerhalb der Wand einen elastischen Abschnitt der Ableitungsleitung (62) zu klammern, um diesen Abschnitt zu schließen, und andererseits den elastischen Abschnitt der Ableitungsleitung (62) offen zu lassen.

15. Maschine nach einem der vorangehenden Ansprüche, **dadurch gekennzeichnet, dass** das Dialysat-Zufuhrsystem (5) einen zusätzlichen Ventrikelbeutel (50') umfasst, wobei die zwei Ventrikelbeutel (50, 50') imstande sind, von den Druckbeaufschlagungsmitteln (70) separat unter Druck gesetzt zu sein.

16. Maschine nach einem der vorangehenden Ansprüche, **dadurch gekennzeichnet, dass** der oder jeder Ventrikelbeutel (50, 50') auch einen Verbindungseingang mit einer Dialysat-Versorgungsquelle (40), vorzugsweise mit einem Versorgungsbeutel größeren Volumens als der oder jeder Ventrikelbeutel (50, 50'), umfasst.

**Claims**

1. A dialysis machine making it possible to treat a body fluid, such as blood or plasma, said machine comprising:

   · a dialyzer (1) including an enclosure housing a dialysis membrane (3) that subdivides the enclosure into a compartment for passing said body fluid and referred to as the "body fluid compartment" (12), and a compartment for passing dialysate and referred to as a "dialysate compartment" (14);
   said body fluid compartment (12) having a body fluid inlet (201) and a body fluid outlet (202), and said dialysate compartment (14) having a dialysate inlet (401) and a dialysate outlet (402);
   · a dialysate feed system (5) comprising a dialysate feed line (52) connected to the inlet (401) of the dialysate compartment (14); and
   · a dialysate discharge system (6) comprising a dialysate discharge line (62) connected to the outlet (402) of the dialysate compartment (14);
   · a control unit (10), such as a programmable controller, that comprises:

      · ultrafiltration control means (UF) making it possible to cause an aqueous quantity of the body fluid to pass through a portion of the membrane (3) from the body fluid compartment (12) into the dialysate compartment (14); and
      · back-filtration control means (RF) making it possible to cause the dialysate to pass through at least a portion of the membrane (3), from the dialysate compartment (14) into the body fluid compartment (12);

   said dialysis machine being **characterized in that** the dialysate feed line (52) is provided with a flow constriction (520), and said machine further comprises means for measuring the pressure difference (523, 524) across the terminals of said flow constriction (520);
   **in that** said dialysate feed system (5) further comprises:

      - at least one flexible bag (50), referred to as a "ventricle bag", designed to contain the dialysate and connected to said feed line (52) upstream from said flow constriction (520), said ventricle bag (50) being contained in an enclosure (500); and
      - pressurizer means for putting the ventricle bag (50) under pressure by pressurizing said enclosure (500);

   and **in that** said machine further comprises an open/close system (C5, C5') for opening/closing the dialysate discharge line (62), which system, when in the open state, allows the dialysate present at the outlet of the dialysate compartment (14) to flow through said discharge line (62), and, when in the closed state, prevents the dialysate present at the outlet of said dialysate compartment (14) from flowing through said discharge line (62).
   said back-filtration control means (RF) being configured to:

      - control said open/close system (C5, C5') so as to close the discharge line (62); and
      - when said discharge line (62) is in the closed state, control the pressure applied to said ventricle bag (50) as a function of the pressure difference measured across the terminals of the constriction (520), so as to obtain a given flow rate of dialysate.

2. A machine according to claim 1, **characterized in that** said dialysate discharge system (6) further comprises at least one discharge bag (60) that is preferably flexible and that has an inlet and an outlet that are connected to the dialysate discharge line (62);
   and **in that** said open/close system (C5, C5') comprises an upstream open/close member (C5) situated between the outlet (402) of the dialysate compartment (14) and said discharge bag (60);

and **in that** said machine further comprises means for determining the quantity of dialysate and of liquid recovered in said discharge bag (60).

3. A machine according to claim 2, **characterized in that** said dialysate discharge system (6) further comprises at least one other discharge bag (60') connected in parallel with said discharge bag (60);
and **in that** said open/close system (C5, C5') further comprises another upstream open/close member (C5') situated between said other discharge bag (60') and the connection inlet node (NE6) for connection to the discharge bags (60, 60');
and **in that** said machine further comprises means for determining the quantity of dialysate and of liquid recovered in said other discharge bag (60').

4. A machine according to claim 2 or claim 3, **characterized in that** said means for determining the quantity of dialysate and of liquid recovered in the discharge bag(s) (60) comprise a flow constriction (620) provided in the dialysate discharge line (62) and situated downstream from said discharge bag(s) (60, 60'), and measurement means for measuring the pressure difference across the terminals of said flow constriction (620).

5. A machine according to any preceding claim, **characterized in that** said means for measuring the pressure difference across the terminals of the constriction (520) in the feed line (52), or, respectively across the terminals of the constriction (620) in the discharge line (62) when said constriction is present, comprise:

· two orifices (517, 518; 617, 618), referred to as "pressure measurement orifices", one of which is provided upstream from said constriction (520; 620) and the other which is provided downstream from said constriction (520; 620) ; and
· for each pressure measurement orifice (517, 518; 617, 618), a pressure sensor (523, 524; 623, 624) arranged in such a manner as to measure the pressure in the corresponding line (52, 62) at said orifice (517, 518; 617, 618) while also being spaced apart from said orifice (517, 518; 617, 618) so as not to be in contact with the dialysate flowing through the line (52, 62).

6. A machine according to claim 5, **characterized in that** a filter (17) that is permeable to air and impermeable to infectious agents and to liquids is interposed between each pressure sensor (523, 524; 623, 624) and the corresponding pressure measurement orifice (517, 518; 617, 618).

7. A machine according to claim 5 or claim 6, **characterized in that** said machine has a frame (8), and said pressure sensors (523, 524, 623, 624) are fastened to the frame of the machine;
and **in that** the dialysate feed line (52) and the dialysate discharge line (62) are suitable for being removed from the remainder of the machine, while the corresponding pressure sensors (523, 524, 623, 624) remain fastened to the frame of the machine.

8. A machine according to any one of claims 4 to 7, taken in combination with claim 3, **characterized in that**, in order to cause the dialysate discharge line (62) to be closed with a view to performing a back-filtration step, said back-filtration control means (RF) are configured to cause each of said upstream open/close members (C5, C5') associated with the discharge bags (60, 60') to close;
and **in that**, in order to cause the dialysate discharge line (62) to be opened with a view to performing an ultrafiltration step, said ultrafiltration control means (UF) are configured to cause one (C5) of said upstream open/close members (C5, C5') to open, and to cause the other (C5') upstream open/close member to close;
said ultrafiltration control means preferably being configured to act, in the next ultrafiltration step, to cause the other (C5') of said upstream open/close members (C5, C5') to open and to cause said upstream open/close member (C5) to close.

9. A machine according to any one of claims 3 to 8, taken in combination with claim 2, **characterized in that** the or each discharge bag (60, 60') is provided with a "downstream" open/close member (C6, C6') situated downstream from said discharge bag (60, 60');
and **in that**, after an ultrafiltration step, said control unit is configured to act, preferably in parallel with a back-filtration step, to cause the downstream open/close member (C6, C6') of the previously filled discharge bag (60, 60') to open in order to discharge its contents;
and, preferably, in order to determine the quantity recovered in said discharge bag (60, 60'), the control unit (10) is configured to measure the flow rate that flows from said discharge bag (60, 60'), using said means for measuring the pressure difference across the terminals of the constriction (620) situated downstream from said discharge bag (60, 60').

10. A machine according to any preceding claim, **characterized in that** said control unit further comprises means for determining an excess aqueous quantity to be removed from the body fluid, which quantity is referred to as the "excess aqueous quantity";

and **in that** the ultrafiltration control means (UF) are configured to extract through the dialysis membrane (3) an aqueous quantity of the body fluid, which quantity is referred to as the "gross aqueous quantity to be removed" and is greater than said excess aqueous quantity that is defined;

the back-filtration control means (RF) being configured to back-filter a quantity of dialysate that is substantially equal to the difference between said gross aqueous quantity to be removed and said excess aqueous quantity.

11. A machine according to any preceding claim, **characterized in that** said control unit (10) is configured to act during a treatment session to cause ultrafiltration steps to take place in alternation with back-filtration steps, the number of alternations preferably being not less than 1000.

12. A machine according to claim 11, **characterized in that**, for a number, referenced N, of cycles, each cycle $i$, where $i$ lies in the range 1 to N, is made up of an ultrafiltration step of duration Te_uf_i and of a back-filtration step of duration Te_rf_i;

said control unit (10) is configured to:

· determine the excess aqueous quantity in the body fluid, referenced Q_aq_excess that is to be removed;
· for each cycle $i$, define the durations Te_uf_i and Te_rf_i, and control the pressurizer means (70) in such a manner that:

```
    Q_aq_excess = Sum over i, where i lies in the range
1 to N, of
K×[(Pa_i +Pv_i)/2 − P14_uf_i]×Te_uf_i − (Dd_rf_i×Te_rf_i)
```

where:

K is a predefined coefficient of ultrafiltration that is specific to the membrane and that expresses the ultrafiltration capacity thereof;
Pa_i is the pressure upstream from the body fluid compartment (12) during the ultrafiltration step of the cycle $i$;
Pv_i is the pressure downstream from the body fluid compartment (12) during the ultrafiltration step of the cycle $i$;
P14_uf_i is the mean pressure in the dialysate compartment (14) during the ultrafiltration step of the cycle $i$; and
Dd_rf_i is the flow rate of dialysate in the dialysate compartment (14) during the back-filtration step of the cycle $i$.

13. A machine according to any preceding claim, **characterized in that** said open/close system (C5, C5') for opening/closing the discharge line (62) comprises at least one clamp suitable firstly for clamping, from the outside, the wall of a flexible portion of said discharge line (62) in such a manner as to close said portion, and secondly for leaving said flexible portion of said discharge line (62) open.

14. A machine according to any one of claims 4 to 12, taken in combination with claim 3, **characterized in that** each of the upstream close/open members (C5, C5'), and, optionally, each of the downstream close/open members (C6, C6'), for closing/opening the discharge line (62) comprises at least one clamp suitable firstly for clamping, from the outside, the wall of a flexible portion of said discharge line (62) in such a manner as to close said portion, and secondly for leaving said flexible portion of said discharge line (62) open.

15. A machine according to any preceding claim, **characterized in that** the dialysate feed system (5) further comprises an additional ventricle bag (50'), the two ventricle bags (50, 50') being suitable for being put under pressure separately by said pressurizer means (70).

16. A machine according to any preceding claim, **characterized in that** said or each ventricle bag (50, 50') is also provided with an inlet for connection to a dialysate feed source (40), preferably a feeder bag that is of larger volume than the or each ventricle bag (50, 50').

FIG.1

EP 2 763 720 B1

523

8

15

16

17

9

521

FIG. 2

EP 2 763 720 B1

FIG. 3

FIG. 4

27

FIG. 5

FIG. 6

EP 2 763 720 B1

FIG. 7

EP 2 763 720 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5011607 A

- US 2009008306 A1